# EUROPEAN PATENT SPECIFICATION

(11) **EP 1 644 485 B1**
(45) Date of publication and mention of the grant of the patent: **04.05.2011**
(21) Application number: 04740821.6
(22) Date of filing: 08.07.2004
(51) Int. Cl.: C12N 5/0735, C12N 5/10

(54) **SECRETED PROTEINS AS MARKERS FOR CELL DIFFERENTIATION**
SEKRETIERTE PROTEINE FÜR ZELLDIFFERENZIERUNGSMARKER
PROTEINES SECRETEES COMME MARQUEURS DE DIFFERENCIATION CELLULAIRE

(30) Priority: 08.07.2003 EP 03015400; 08.07.2003 US 485462 P
(43) Date of publication of application: 12.04.2006
(73) Proprietor: AXIOGENESIS AG, 50829 Köln (DE)
(72) Inventor: EHLICH, Andreas, 53894 Mechernich (DE); BOHLEN, Heribert, 50733 Köln (DE); SCHWENGBERG, Silke, 52355 Düren (DE)
(74) Representative: Steinecke, Peter
(86) International application number: PCT/EP2004/007529
(87) International publication number: WO 2005/005662

(56) References cited:
- WO-A-95/14079
- WO-A-98/49320
- WO-A-02/051987
- US-A1- 2002 042 096
- US-A1- 2003 008 836
- MUELLER M ET AL: "Selection of ventricular-like cardiomyocytes from ES cells in vitro" FASEB JOURNAL, FED. OF AMERICAN SOC. FOR EXPERIMENTAL BIOLOGY, BETHESDA, MD, US, vol. 14, no. 15, December 2000 (2000-12), pages 2540-2548, XP002180458 ISSN: 0892-6638
- WOBUS A M ET AL: "RETINOIC ACID INDUCES EXPRESSION OF THE VENTRICULAR 2.1 KB MYOSIN-LIGHT-CHAIN-2 PROMOTER DURING IN VITRO CARDIOGENESIS OF EMBRYONIC STEM CELLS" CIRCULATION, AMERICAN HEART ASSOCIATION, DALLAS, TX, US, vol. 92, no. 8, SUPPL 1, 15 October 1995 (1995-10-15), page I114, XP002001047 ISSN: 0009-7322
- WOBUS A M ET AL: "RETINOIC ACID ACCELERATES EMBRYONIC STEM CELL-DERIVED CARDIAC DIFFERENTATION AND ENHANCES DEVELOPMENT OF VENTRICULAR CARDIOMYOCYTES" JOURNAL OF MOLECULAR AND CELLULAR CARDIOLOGY, XX, XX, vol. 29, no. 6, 1997, pages 1525-1539, XP001030714 ISSN: 0022-2828
- KLUG M G ET AL: "GENETICALLY SELECTED CARDIOMYOCYTES FROM DIFFERENTIATING EMBRYONIC STEM CELLS FORM STABLE INTRACARDIAC GRAFTS" JOURNAL OF CLINICAL INVESTIGATION, NEW YORK, NY, US, vol. 98, no. 1, 1 July 1996 (1996-07-01), pages 216-224, XP000670716 ISSN: 0021-9738
- BRONSTEIN I ET AL: "CHEMILUMINESCENT REPORTER GENE ASSAYS: SENSITIVE DETECTION OF THE GUS AND SEAP GENE PRODUCTS" BIOTECHNIQUES, EATON PUBLISHING, NATICK, US, vol. 17, no. 1, 1994, pages 172-174,176-177, XP009011110 ISSN: 0736-6205
- HUNTER J J ET AL: "VENTRICULAR EXPRESSION OF A MLC-2V-RAS FUSION GENE INDUCES CARDIAC HYPERTROPHY AND SELECTIVE DIASTOLIC DYSFUNCTION IN TRANSGENIC MICE" JOURNAL OF BIOLOGICAL CHEMISTRY, AMERICAN SOCIETY OF BIOLOGICAL CHEMISTS, BALTIMORE, MD, US, vol. 270, no. 39, 1 September 1995 (1995-09-01), pages 23173-23178, XP002001049 ISSN: 0021-9258
- LIU HSIAO-SHENG ET AL: "Is green fluorescent protein toxic to the living cells?" BIOCHEMICAL AND BIOPHYSICAL RESEARCH COMMUNICATIONS, vol. 260, no. 3, 14 July 1999 (1999-07-14), pages 712-717, XP002257794 ISSN: 0006-291X cited in the application
- GUAN K. ET AL.: "Embryonic stem cells in vitro - Prospects for cell and developmental biology, embryotoxicology and cell therapy" ALTEX, vol. 16, no. 3, 1999, pages 135-141, XP009018838 cited in the application
- METZGER JOSEPH M ET AL: "Vital staining of cardiac myocytes during embryonic stem cell cardiogenesis in vitro" CIRCULATION RESEARCH, GRUNE AND STRATTON, BALTIMORE, US, vol. 78, no. 4, 1996, pages 547-552, XP002213275 ISSN: 0009-7330 cited in the application
- ROHWEDEL J ET AL: "EMBRYONIC STEM CELLS AS AN IN VITRO MODEL FOR MUTAGENICITY, CYTOTOXICITY AND EMBRYOTOXICITY STUDIES: PRESENT STATE AND FUTURE PROSPECTS" TOXICOLOGY IN VITRO, ELSEVIER SCIENCE, GB, vol. 15, no. 6, 2001, pages 741-753, XP001027083 ISSN: 0887-2333

### SEQUENCE LISTING

<110> Axiogenesis AG
<120> Secreted Proteins As Markers For Cell Differentiation
<130> AX02A03/P-WO
<160> 4
<170> PatentIn version 3.1
<210> 1
   <211> 5443
   <212> DNA
   <213> Mus musculus
<220>
   <221> promoter
   <222> (1)..(5443)
   <223> alpha myosin heavy chain gene, promoter region
<220>
   <221> promoter
   <222> (2)..(5442)
   <223> promoter region contained in SEQ ID NO 3
<220>
   <221> 5'UTR
   <222> (4369)..(4389)
   <223> exon1
<220>
   <221> 5'UTR
   <222> (5071)..(5139)
   <223> exon 2
<220>
   <221> 5'UTR
   <222> (5431)..(5443)
   <223> exon 3
<400> 1
   ggatcctgca aggtcacaca agggtctcca cccaccaggt gccctagtct caatttcagt     60
   ttccatgcct tgttctcaca atgctggcct ccccagagct aatttggact ttgtttttat    120
   ttcaaaaggg cctgaatgag gagtagatct tgtgctaccc agctctaagg gtgcccgtga    180
   agccctcaga cctggagcct ttgcaacagc cctttaggtg gaagcagaat aaagcaattt    240
   tccttaaagc caaaatcctg cctctagact cttcttctct gacctcggtc cctgggctct    300
   agggtgggga ggtggggctt ggaagaagaa ggtggggaag tggcaaaagc cgatccctag    360
   ggccctgtga agttcggagc cttccctgta cagcactggc tcatagatcc tcctccagcc    420
   aaacatagca agaagtgata cctcctttgt gacttcccca ggcccagtac ctgtcaggtt    480
   gaaacaggat ttagagaagc ctctgaactc acctgaactc tgaagctcat ccaccaagca    540
   agcacctagg tgccactgct agttagtatc ctacgctgat aatatgcaga gctgggccac    600
   agaagtcctg gggtgtagga actgaccagt gacttttcag tcggcaaagg tatgaccccc    660
   tcagcagatg tagtaatgtc cccttagatc ccatcccagg caggtctcta agaggacatg    720
   ggatgagaga tgtagtcatg tggcattcca aacacagcta tccacagtgt cccttgcccc    780
   ttccacttag ccaggaggac agtaacctta gcctatcttt cttcctcccc atcctcccag    840
   gacacacccc ctggtctgca gtattcattt cttccttcac gtcccctctg tgacttccat    900
   ttgcaaggct tttgacctct gcagctgctg gaagatagag tttggcccta ggtgtggcaa    960
   gccatctcaa gagaaagcag acaacagggg gaccagattt tggaaggatc aggaactaaa   1020
   tcactggcgg gcctgggggt agaaaaaaga gtgagtgagt ccgctccagc taagccaagc   1080
   tagtccccga gatactctgc cacagctggg ctgctcgggg tagctttagg aatgtgggtc   1140
   tgaaagacaa tgggattgga agacatctct ttgagtctcc cctcaacccc acctacagac   1200
   acactcgtgt gtggccagac tcctgttcaa cagccctctg tgttctgacc actgagctag   1260
   gcaaccagag catgggccct gtgctgagga tgaagagttg gttaccaata gcaaaaacag   1320
   caggggaggg agaacagaga acgaaataag gaaggaagaa ggaaaggcca gtcaatcaga   1380
   tgcagtcaga agagatggga agccaacaca cagcttgagc agaggaaaca gaaaagggag   1440
   agattctggg cataaggagg ccacagaaag aagagcccag gccccccaag tctcctcttt   1500
   ataccctcat cccgtctccc aattaagccc actcttcttc ctagatcaga cctgagctgc   1560
   agcgaagaga cccgtaggga ggatcacact ggatgaagga gatgtgtgga gaagtccagg   1620
   gcaacctaag agccagagcc taaaagagca agagataaag gtgcttcaaa ggtggccagg   1680
   ctgtgcacac agagggtcga ggactggtgg tagagcctca agataaggat gatgctcaga   1740
   atgggcgggg ggggggattc tggggggggg agagagaagg tgagaaggag cctggaacag   1800
   agaatctgga agcgctggaa acgataccat aaagggaaga acccaggcta cctttagatg   1860
   taaatcatga aagacaggga gaagggaagc tggagagagt agaaggaccc cggggcaaga   1920
   catggaagca aggacaagcc aggttgagcg ctccgtgaaa tcagcctgct gaaggcagag   1980
   ccctggtatg agcaccagaa cagcagaggc tagggttaat gtcgagacag ggaacagaag   2040
   gtagacacag gaacagacag agacggggga gccaggtaac aaaggaatgg tccttctcac   2100
   ctgtggccag agcgtccatc tgtgtccaca tactctagaa tgttcatcag actgcagggc   2160
   tggcttggga ggcagctgga aagagtatgt gagagccagg ggagacaagg gggcctagga   2220
   aaggaagaag agggcaaacc aggccacaca agagggcaga gcccagaact gagttaactc   2280
   cttccttgtt gcatcttcca taggaggcag tgggaactct gtgaccacca tcccccatga   2340
   gcccccacta cccataccaa gtttggcctg agtggcattc taggttccct gaggacagag   2400
   cctggccttt gtctcttgga cctgacccaa gctgacccaa tgttctcagt accttatcat   2460
   gccctcaaga gcttgagaac caggcagtga catattaggc catgggctaa ccctggagct   2520
   tgcacacagg agcctcaagt gacctccagg gacacagctg cagacaggtg gcctttatcc   2580
   ccaaagagca accatttggc ataggtggct gcaaatggga atgcaaggtt gaatcaggtc   2640
   ccttcaagaa tactgcatgc aagacctaag acccctggag agaggggtat gctcctgccc   2700
   ccacccacca taaggggagt gaactatcct agggggctgg cgaccttggg gagacaccac   2760
   attactgaga gtgctgagcc cagaaaaact gaccgccctg tgtcctgccc acctccacac   2820
   tctagagcta tattgagagg tgacagtaga tagggtggga gctggtagca gggagagtgt   2880
   tcctgggtgt gagggtgtag gggaaagcca gagcagggga gtctggcttt gtctcctgaa   2940
   cacaatgtct acttagttat aacaggcatg acctgctaaa gacccaacat ctacgacctc   3000
   tgaaaagaca gcagccctgg aggacagggg ttgtctctga gccttgggtg cttgatggtg   3060
   ccacaaagga gggcatgagt gtgagtataa ggccccagga gcgttagaga agggcacttg   3120
   ggaaggggtc agtctgcaga gcccctatcc atggaatctg gagcctgggg ccaactggtg 3180
   taaatctctg ggcctgccag gcattcaaag cagcacctgc atcctctggc agcctgggga 3240
   ggcggaaggg agcaaccccc cacttatacc ctttctccct cagccccagg attaacacct 3300
   ctggccttcc cccttcccac ctcccatcag gagtggaggg ttgcagaggg agggtaaaaa 3360
   cctacatgtc caaacatcat ggtgcacgat atatggatca gtatgtgtag aggcaagaaa 3420
   ggaaatctgc aggcttaact gggttaatgt gtaaagtctg tgtgcatgtg tgtgtgtctg 3480
   actgaaaacg ggcatggctg tgcagctgtt cagttctgtg cgtgaggtta ccagactgca 3540
   ggtttgtgtg taaattgccc aaggcaaagt gggtgaatcc cttccatggt ttaaagagat 3600
   tggatgatgg cctgcatctc aaggaccatg gaaaatagaa tggacactct atatgtgtct 3660
   ctaagctaag gtagcaaggt ctttggagga cacctgtcta gagatgtggg caacagagac 3720
   tacagacagt atctgtacag agtaaggaga gagaggaggg ggtgtagaat tctcttacta 3780
   tcaaagggaa actgagtcgt gcacctgcaa agtggatgct ctccctagac atcatgactt 3840
   tgtctctggg gagccagcac tgtggaactt caggtctgag agagtaggag gctcccctca 3900
   gcctgaagct atgcagatag ccagggttga aagggggaag ggagagcctg ggatgggagc 3960
   ttgtgtgttg gaggcagggg acagatatta agcctggaag agaaggtgac ccttacccag 4020
   ttgttcaact cacccttcag attaaaaata actgaggtaa gggcctgggt aggggaggtg 4080
   gtgtgagacg ctcctgtctc tcctctatct gcccatcggc cctttgggga ggaggaatgt 4140
   gcccaaggac taaaaaaagg ccatggagcc agaggggcga gggcaacaga cctttcatgg 4200
   gcaaaccttg gggccctgct gtcctcctgt cacctccaga gccaagggat caaaggagga 4260
   ggagccagga caggagggaa gtgggaggga gggtcccagc agaggactcc aaatttaggc 4320
   agcaggcata tgggatggga tataaagggg ctggagcact gagagctgtc agagatttct 4380
   ccaacccagg taagagggag tttcgggtgg gggctcttca cccacaccag acctctcccc 4440
   acctagaagg aaactgcctt tcctggaagt ggggttcagg ccggtcagag atctgacagg 4500
   gtggccttcc accagcctgg gaagttctca gtggcaggag gtttccacaa gaaacactgg 4560
   atgccccttc ccttacgctg tcttctccat cttcctcctg ggatgctccc tccccgtctt 4620
   ggtttatctt ggctcttcgt cttcagcaag atttgccctg tgctgtccac tccatctttc 4680
   tctactgtct ccgtgccttg ccttgccttc ttgcgtgtcc ttcctttcca cccatttctc 4740
   acttcacctt ttctcccctt ctcatttgta ttcatccttc cttccttcct tccttccttc 4800
   cttccttcct tccttccttc ctttctccct tccttccttc cttccttcct tccttccttc 4860
   cttccttcct gtgtcagagt gctgagaatc acacctgggg ttcccaccct tatgtaaaca 4920
   atcttccagt gagccacagc ttcagtgctg ctgggtgctc tcttaccttc ctcaccccct 4980
   ggcttgtcct gttccatcct ggtcaggatc tctagattgg tctcccagcc tctgctactc   5040
   ctcttcctgc ctgttcctct ctctgtccag ctgcgccact gtggtgcctc gttccagctg   5100
   tggtccacat tcttcaggat tctctgaaaa gttaaccagg tgagaatgtt tcccctgtag   5160
   acagcagatc acgattctcc cggaagtcag gcttccagcc ctctctttct ctgcccagct   5220
   gcccggcact cttagcaaac ctcaggcacc cttaccccac atagacctct gacagagaag   5280
   caggcacttt acatggagtc ctggtgggag agccataggc tacggtgtaa aagaggcagg   5340
   gaagtggtgg tgtaggaaag tcaggacttc acatagaagc ctagcccaca ccagaaatga   5400
   cagacagatc cctcctatct cccccataag agtttgagtc gac                     5443
<210> 2
   <211> 6298
   <212> DNA
   <213> Mus musculus
<220>
   <221> promoter
   <222> (1)..(5445)
   <223> ventricular myosin regulatory light chain gene (AF302688)
<220>
   <221> 5'UTR
   <222> (5446)..(5486)
   <223> exon 1
<220>
   <221> exon
   <222> (5487)..(5489)
   <223> exon 1
<220>
   <221> exon
   <222> (6209)..(6298)
   <223> exon 2
<400> 2
   ctcgagtgct gggattaaag gcatgcacca ccatgctcag cctgttttct tttttaagag      60
   ttaatctttt taattatgtg tatgtatgtc tttgcatgga tatgcccacg agagtgcatg     120
   tgccctggaa gaccagaagg gggtgacaaa cccctgcagt tttgagcctc ctggcctggg     180
   tgctgggaat caaacttaat ttctctagaa gagcagagtc ctcttaacac ccaagccctc     240
   ttgccagccc ttattttgtt tggttggttg gtttttgcca aagctaggag ttccagcccc     300
   agtcttttat actctgccct catggcttct gacatctcca actctgccag gggctgactc      360
   ttatcttcag aaagcaattg ttactttgac ccccagcagt gctgagccac cacccagggc     420
   ctgaggctag aagaaaatgt accctccttc catggtcatc ttcagaaagc actgtgaacc     480
   caggtaaagg tagcaggtcc aggttcagat gtagtgtcca cagcctgtgc aaaaaggacc     540
   cttcacttcc aagcttgccc cacctgatca gaccactctc cagatctggg ggaaacggta     600
   gcaggctctt caccccaaca ggagtcccca gggcacacaa gatattgcct ttgattttgt     660
   ccttatgaca accctgcgaa taaaatcgtc atcatgccca ctctacagat tagaatactg     720
   aagctgagag caaaaaccta cctgatgtca cactgctgct caatgactaa gcaacgaaaa     780
   tcaaaagtga atattcccgc aggctccagt cttgcagatg acaaatgctc tgtcaccgtc     840
   acatttgggt ccatgttctg aaatcctctg ttttcacctc cctagaaccc cagatagtac     900
   ctaaatagct gagtccattc acctggccct ggctttctgc taaggaatat gtcagacatc     960
   ttcaggattc ttcaaacttg ttctgagtag aaatctcttc aggcctggga gctgtttcct    1020
   ctcagcagaa agcccccatg tggcacaggc caactccccc acaaccagaa ccatcatcag    1080
   tcacaactgc acttcctctc atgccgaaga aacttttttt ttcagtcttg gatggatgct   1140
   ggaaccatcg gatcctgtaa cccgcttctc cacctccagt acagacgtca ggacttgagg    1200
   ctccctccgt ctgctctctt ctgcactgca cttgaatttc cctgaggttt ggcatttggg    1260
   gtttttgttt ggcttgtttc tttgcttgcc tagaggaata atcaatgctt aggcttttaa    1320
   tagcaaaggc atttagtggg tcaaccaagc actcaggaga tagacaggag ggtcagaatt    1380
   caaggtcatc ctttgctaca caatgagttc agggtcagcc tgggctacac gagatccaat    1440
   ctaaaataaa caaaaaataa aataattgat atttcttctc tgtatattat agttatactg    1500
   tagactgttg gggatacatt ctccaagagt ctcaaagttc atctccactg agttaattaa    1560
   tgttgttact ataacacaac actagagtct ggttcgttta caaatacaaa agatttattt    1620
   tgggctggag agatggctca gagctgactg ctcttccaaa ggtcctgagt tccaatccca    1680
   gcaaccacat ggtggctcga aaccatctgt aatgggatct gatgccttct tctagtgtgt    1740
   ctgaagacag ctatggtgta ctcacataca taaaataaat aaataaatct taagagagag 1800
   atttgtttct acaaaggcta gatgctactg agagtaaagg gtttagaact ccgttttcct 1860
   cttcttacta actcactaat cccatcttgg gggggggagg gggaagcttc atgacttcac 1920
   ctaacctcgt tcacctctcc tagaccttac ctcctatcgc taccaatctc tgatttgggg 1980
   atcaaatttt caacccagga gctttaggga gacaaatgta aaccccagta tcccctgact 2040
   cccggtgtac actggatcct gcacacagta gggctccaat aaatactgaa agtccttaga 2100
   atgggggcaa aatcattttt gtgtttataa atatgaccca catgttctct catcttttaa 2160
   aattgtcaga gtaatttctc tctctctctc tctctctctc tctgtgtgtg tgtgtgtgtc 2220
   tgtgtctgtg tgtgtgtctg tgtctgtgtc tgtgtgtctg tgtgtgtgta ctcacatcca 2280
   agtgggtttg ggtacctgtc atgtggacat gtatatgttg gtagaagcca gaagtcaacc 2340
   ttgtgtgtca tttctcagag gtgatccatc ttattttttg agacagggtc tctcactgag 2400
   gccggggact tactgttttt aggttacacc tgctggccag caagccagag agaccgagag 2460
   agacggtctg tctccatctc tccagcgaca ggctttcaga cccacaccac catgcccagc 2520
   tttgatgtgg gtctgggttt tgaggagtag aattcaggac ctcttgctga gccatctccc 2580
   cagccactga acataattca tatataatct ggcttttgtt ccttttgtgg gtgcagggtg 2640
   tgttttgttt tgttctttgc tgtgtattat tctgtgcatt taagcttttt ttttttgtga 2700
   ctacctggga gggacactga acagccagaa aggccaggcc gaggcttcac tgatggggat 2760
   gtgagcccgg gaatgctggc agctgccggg agctggaaag ggcaaaggaa aggactgtct 2820
   ccgcatccac ggtggacgca gccctctgcc cgatttctat ttctggccac cagctctgcc 2880
   aggtagcgag cttggctgct ctgagctgcc tgggtttgcc gttgtatttt cctactagca 2940
   tgggaaagcg tgatcagctt gctttgttct caattgttcc agaagctctg ccggtcccct 3000
   ccaggactcc tgagtctctg ctccgtggta ctcggggctg gctctcaaag ttctaggctg 3060
   cagaaatctc acaagcgcat gagcttagag tcccagggtg gatgtccacc aagagcccag 3120
   ggacaaagca ttgacagctc ctgtgtgcgc cacgtctccc cccaccccca cccctacccc 3180
   caggaactgt gagaggagtg cagagcccct cccccaggcc tttccaacaa ggactcctgg 3240
   aggacccttg ggtttttaac accaaatcac caaatgtttc ccacgcagca acacaaacca 3300
   gctcttcctc atacagcacg gtgggccagt ggaccatggg gacaggttac ctctgtgggc 3360
   ccaggctcac ggtaaactct aacctcaatc tgtagcctcc cacagccatt tgcgggtcac 3420
   cttgcttctc agccaccgtg tggcacttgg caagtcacgt gtgcctcaac acaataagaa 3480
   gccaagggaa taggggcttt gcttaactgg tacagcagtg tagcccaagc tagccttgaa 3540
   ctcactatgt agccaaggac gatcataaac tcttgatcct cccgcttcag agtcctgggt 3600
   gttgagataa caggtgtggg tcactcccta cccttcttct aatagcaatc aatgtgtggc 3660
   cacatgtttg tgcctcacag attaaaacca tcttgacctg aggacgaaat gactaacagt   3720
   tgcctcctga aggttgcctg gatctcatct ttataatccc agcaatcaag gggagtgggg   3780
   gatcaggagt tcaaagtcag cccagcctgg gctacatgag accctgtctt gaaaaatgga   3840
   ggaattaagc tgggcgtggt gccgcactcc tttaatccca gcacttggga ggtagaggca   3900
   ggcggacttc tgagttcgag gccagcctgg tctacaaagt gagttccagg acagccacag   3960
   ctatacagag aaaccctgtc tcgaaaaacc agaaagaaag aaagagagaa agaaagaaag   4020
   aaagaaagaa agaaagaaag aaagaaagga aggaaggaag gaaggaagga aaggaaggaa   4080
   ggaaggaagg aaagaaagaa agaaagaaag aaagaaagac agacagaaag aaaggttagg   4140
   aaagaaagaa aggaaaagaa agaaagaaag aaagaaagaa agaaagaaag aaagaaagaa   4200
   agacagaaag aaaggtttgg aaagaaagaa aggaaaagaa agaaagaaag agagaaagag   4260
   agaaagaaag aaagaaaaga aaagagagaa aagaaaagaa aagaaaagaa aagaaagaaa   4320
   agaaaaaaaa gaaaagaaaa gaaagaaaag aaaaggaagg aagaaaagaa aagaaaaatg   4380
   gaggagttaa ccctatgttt cctttttttt tattcatcat tggtgaggct atcctcagct   4440
   acatatcaag ttcaagccag cctgggctac atgagaccct gcctcaaaaa agaaaaggag   4500
   ccagtgtagc gacatactcc cgtcctccca gcacttggga gacagaggct actccactgc   4560
   tgtctccagc agccggcctg cctccctgag cctcattttt ttcataacat ggggacccaa   4620
   ctgctaaggt gaccttgctc ccatggggtg actggagact tgagagtgaa gtggttatca   4680
   tttgtccagt ctgtgaacaa atggcagcct ccaaggtggt tttgtgttca aaggaggaca   4740
   tgggacaggg agaggccagg gagaagagcc caccctcagg agtaggctgt ccccgtgaag   4800
   ctgggtgggg acaaaaagca gagaagcaga ggcagaggac aagcgtgggt gacatttgag   4860
   caaagatggg aatgtgccag aggctgccca agatgtgcat gtgcaaaggc cctgaggtgc   4920
   aagggtgcct ggatccagag ccaaaagctc aggctccctc ctcctcttcc tcctcttcct   4980
   cctcttcctc ctctttctcc tctccctcct ctccctcctc ccccttcctc ttcctctccc   5040
   cttcctcctc ttccttctcc ccttccttct cctccccttc ctcctcctcc tcctcctgct   5100
   cctcctcctc ctcctcctgc tcctcctcct gctcctcctc ctcctcctcc tgggttactc   5160
   ttccccatta gacaatggca gggaagagag cacaccccat catccccagg ccaggcccca   5220
   gccactgact ctttaacctt gaaggcattt ttgggtctca cgtgtccacc caggcgggtg   5280
   gccgcctttg agcagctctt acttcagaag aacggcatgg agtggggggt ggggggctta   5340
   ggtggcctcc gcctcaccta caactgccaa aagtggtcat ggggttattt ttaaccccag   5400
   gggagaggta tttattgttc cacagcaggg gcagaggcca gcaggctcct cgaactctcc   5460
   agaggtggca actggcctca gacacc atg gtgagtggtc agtaaaccct tgagaagaga   5519
                                Met
   1
   cgcagggtgg ggagcagaga gataaccccc tcccaagcca agcaccccat ggaggagggg   5579
   gggaggagga ggggaaggag gagggaagct ctcttacgag ccccctagcc ctagatggac   5639
   cagcaccttg caccctctga gggaccccaa tcagctcccc taaggagcca caaatagcag   5699
   ctcctcaagg aacttgcaaa aatcaatgag aaatgcgctt gggggatggg tgcccactac   5759
   ctgatctcaa gaaatcagta acaccccacc cccaccccac ccagagcttg ccaaacggag   5819
   actgagagct tttaagggtc gaattgtaat tcttttccca attcaggtgg ccaggaagag   5879
   gttttctggt tctctctttt gaatattccc cttgaaatat ttgtgcccgc ctcccagaac   5939
   aggtagcccc cagctgctag agactgcagc taaggggcgc agagtgtacg tgtgtgtggc   5999
   tgtgtgatct agagaagtga ctcaccctct ctgagcctcc agtctcctta gtggagcaga   6059
   ggagagcatt agataatgtt tggaggtttt ggggtatcat ttcgcctcgc atgttgtctg   6119
   ggtaccagag actcactccc caggtgacag gtcctggccc aggtcctgat ccagaggctc   6179
   cacagtgtct gatggatatt cctctccag gca cca aag aaa gcc aag aag cgg     6232
                                   Ala Pro Lys Lys Ala Lys Lys Arg
                                               5
   ata gaa ggc ggg agc tcc aac gtg ttc tcc atg ttt gag cag acc cag     6280
   Ile Glu Gly Gly Ser Ser Asn Val Phe Ser Met Phe Glu Gln Thr Gln
   10                  15                  20                  25
   atc cag gag ttc aag gaa                                             6298
   Ile Gln Glu Phe Lys Glu
                       30
<210> 3
   <211> 10475
   <212> DNA
   <213> Artificial Sequence
<220>
   <223> composite vector
   <220>
   <221> promoter
   <222> (77)..(5517)
   <223> 77-5517 Mus musculus alpha myosin heavy chain gene, promoter regi on (Acc. No. U71441)
<220>
   <221> CDS
   <222> (5558)..(7117)
   <223> SEAP
<220>
   <221> rep_origin
   <222> (9750)..(10393)
   <223> pUC plasmid replication origin
<220>
   <221> polyA_signal
   <222> (9401)..(9406)
   <223> Herpes simplex virus thymidine kinase polyadenylation signal
<220>
   <221> polyA_signal
   <222> (9414)..(9419)
   <223> Herpes simplex virus thymidine kinase polyadenylation signal
<220>
   <221> terminator
   <222> (9163)..(9165)
   <223> Kanamycin/neomycin resistance gene
<220>
   <221> ATG
   <222> (8371)..(8373)
   <223> Kanamycin/neomycin resistance gene
<220>
   <221> promoter
   <222> (8242)..(8248)
   <223> SV40 early promoter element
<220>
   <221> repeat_region
   <222> (8167)..(8230)
   <223> SV40 early promoter
<220>
   <221> enhancer
   <222> (8018)..(8163)
   <223> SV40 early promoter
<220>
   <221> rep_origin
   <222> (8187)..(8322)
   <223> SV40 origin of replication
<220>
   <221> -10_signal
   <222> (7931) .. (7936)
   <223> Ampicillin resistance (b-lactamase) promoter
<220>
   <221> -35_signal
   <222> (7908)..(7913)
   <223> Ampicillin resistance (b-lactamase) promoter
<220>
   <221> polyA_signal
   <222> (7294) .. (7299)
   <223> SV40 early mRNA polyadenylation signal
<220>
   <221> polyA_signal
   <222> (7323)..(7328)
   <223> SV40 early mRNA polyadenylation signal
<220>
   <221> mature protein
   <222> (5609) .. (7114)
   <223> SEAP
<220>
   <221> start codon
   <222> (5558)..(5560)
   <223> ATG of the Secreted alkaline phosphatase (SEAP) gene
<220>
   <221> sig_peptide
   <222> (5558)..(5608)
   <223> SEAP
<400> 3
   tagttattac tagcgctacc ggactcagat ctcgagctca agcttcgaat tctgcagtcg      60
   acggtaccgc gggcccgatc ctgcaaggtc acacaagggt ctccacccac caggtgccct     120
   agtctcaatt tcagtttcca tgccttgttc tcacaatgct ggcctcccca gagctaattt     180
   ggactttgtt tttatttcaa aagggcctga atgaggagta gatcttgtgc tacccagctc     240
   taagggtgcc cgtgaagccc tcagacctgg agcctttgca acagcccttt aggtggaagc     300
   agaataaagc aattttcctt aaagccaaaa tcctgcctct agactcttct tctctgacct     360
   cggtccctgg gctctagggt ggggaggtgg ggcttggaag aagaaggtgg ggaagtggca     420
   aaagccgatc cctagggccc tgtgaagttc ggagccttcc ctgtacagca ctggctcata     480
   gatcctcctc cagccaaaca tagcaagaag tgatacctcc tttgtgactt ccccaggccc 540
   agtacctgtc aggttgaaac aggatttaga gaagcctctg aactcacctg aactctgaag 600
   ctcatccacc aagcaagcac ctaggtgcca ctgctagtta gtatcctacg ctgataatat 660
   gcagagctgg gccacagaag tcctggggtg taggaactga ccagtgactt ttcagtcggc 720
   aaaggtatga ccccctcagc agatgtagta atgtcccctt agatcccatc ccaggcaggt 780
   ctctaagagg acatgggatg agagatgtag tcatgtggca ttccaaacac agctatccac 840
   agtgtccctt gccccttcca cttagccagg aggacagtaa ccttagccta tctttcttcc 900
   tccccatcct cccaggacac accccctggt ctgcagtatt catttcttcc ttcacgtccc 960
   ctctgtgact tccatttgca aggcttttga cctctgcagc tgctggaaga tagagtttgg 1020
   ccctaggtgt ggcaagccat ctcaagagaa agcagacaac agggggacca gattttggaa 1080
   ggatcaggaa ctaaatcact ggcgggcctg ggggtagaaa aaagagtgag tgagtccgct 1140
   ccagctaagc caagctagtc cccgagatac tctgccacag ctgggctgct cggggtagct 1200
   ttaggaatgt gggtctgaaa gacaatggga ttggaagaca tctctttgag tctcccctca 1260
   accccaccta cagacacact cgtgtgtggc cagactcctg ttcaacagcc ctctgtgttc 1320
   tgaccactga gctaggcaac cagagcatgg gccctgtgct gaggatgaag agttggttac 1380
   caatagcaaa aacagcaggg gagggagaac agagaacgaa ataaggaagg aagaaggaaa 1440
   ggccagtcaa tcagatgcag tcagaagaga tgggaagcca acacacagct tgagcagagg 1500
   aaacagaaaa gggagagatt ctgggcataa ggaggccaca gaaagaagag cccaggcccc 1560
   ccaagtctcc tctttatacc ctcatcccgt ctcccaatta agcccactct tcttcctaga 1620
   tcagacctga gctgcagcga agagacccgt agggaggatc acactggatg aaggagatgt 1680
   gtggagaagt ccagggcaac ctaagagcca gagcctaaaa gagcaagaga taaaggtgct 1740
   tcaaaggtgg ccaggctgtg cacacagagg gtcgaggact ggtggtagag cctcaagata 1800
   aggatgatgc tcagaatggg cggggggggg gattctgggg gggggagaga gaaggtgaga 1860
   aggagcctgg aacagagaat ctggaagcgc tggaaacgat accataaagg gaagaaccca 1920
   ggctaccttt agatgtaaat catgaaagac agggagaagg gaagctggag agagtagaag 1980
   gaccccgggg caagacatgg aagcaaggac aagccaggtt gagcgctccg tgaaatcagc 2040
   ctgctgaagg cagagccctg gtatgagcac cagaacagca gaggctaggg ttaatgtcga 2100
   gacagggaac agaaggtaga cacaggaaca gacagagacg ggggagccag gtaacaaagg 2160
   aatggtcctt ctcacctgtg gccagagcgt ccatctgtgt ccacatactc tagaatgttc 2220
   atcagactgc agggctggct tgggaggcag ctggaaagag tatgtgagag ccaggggaga 2280
   caagggggcc taggaaagga agaagagggc aaaccaggcc acacaagagg gcagagccca 2340
   gaactgagtt aactccttcc ttgttgcatc ttccatagga ggcagtggga actctgtgac 2400
   caccatcccc catgagcccc cactacccat accaagtttg gcctgagtgg cattctaggt 2460
   tccctgagga cagagcctgg cctttgtctc ttggacctga cccaagctga cccaatgttc 2520
   tcagtacctt atcatgccct caagagcttg agaaccaggc agtgacatat taggccatgg 2580
   gctaaccctg gagcttgcac acaggagcct caagtgacct ccagggacac agctgcagac 2640
   aggtggcctt tatccccaaa gagcaaccat ttggcatagg tggctgcaaa tgggaatgca 2700
   aggttgaatc aggtcccttc aagaatactg catgcaagac ctaagacccc tggagagagg 2760
   ggtatgctcc tgcccccacc caccataagg ggagtgaact atcctagggg gctggcgacc 2820
   ttggggagac accacattac tgagagtgct gagcccagaa aaactgaccg ccctgtgtcc 2880
   tgcccacctc cacactctag agctatattg agaggtgaca gtagataggg tgggagctgg 2940
   tagcagggag agtgttcctg ggtgtgaggg tgtaggggaa agccagagca ggggagtctg 3000
   gctttgtctc ctgaacacaa tgtctactta gttataacag gcatgacctg ctaaagaccc 3060
   aacatctacg acctctgaaa agacagcagc cctggaggac aggggttgtc tctgagcctt 3120
   gggtgcttga tggtgccaca aaggagggca tgagtgtgag tataaggccc caggagcgtt 3180
   agagaagggc acttgggaag ggtcagtctg gcagagcccc tatccatgga atctggagcc 3240
   tggggccaac tggtgtaaat ctctgggcct gccaggcatt caaagcagca cctgcatcct 3300
   ctggcagcct ggggaggcgg aagggagcaa ccccccactt ataccctttc tccctcagcc 3360
   ccaggattaa cacctctggc cttccccctt cccacctccc atcaggagtg gagggttgca 3420
   gagggagggt aaaaacctac atgtccaaac atcatggtgc acgatatatg gatcagtatg 3480
   tgtagaggca agaaaggaaa tctgcaggct taactgggtt aatgtgtaaa gtctgtgtgc 3540
   atgtgtgtgt gtctgactga aaacgggcat ggctgtgcag ctgttcagtt ctgtgcgtga 3600
   ggttaccaga ctgcaggttt gtgtgtaaat tgcccaaggc aaagtgggtg aatcccttcc 3660
   atggtttaaa gagattggat gatggcctgc atctcaagga ccatggaaaa tagaatggac 3720
   actctatatg tgtctctaag ctaaggtagc aaggtctttg gaggacacct gtctagagat 3780
   gtgggcaaca gagactacag acagtatctg tacagagtaa ggagagagag gagggggtgt 3840
   agaattctct tactatcaaa gggaaactga gtcgtgcacc tgcaaagtgg atgctctccc 3900
   tagacatcat gactttgtct ctggggagcc agcactgtgg aacttcaggt ctgagagagt 3960
   aggaggctcc cctcagcctg aagctatgca gatagccagg gttgaaaggg ggaagggaga 4020
   gcctgggatg ggagcttgtg tgttggaggc aggggacaga tattaagcct ggaagagaag 4080
   gtgaccctta cccagttgtt caactcaccc ttcagattaa aaataactga ggtaagggcc 4140
   tgggtagggg aggtggtgtg agacgctcct gtctctcctc tatctgccca tcggcccttt 4200
   ggggaggagg aatgtgccca aggactaaaa aaaggccatg gagccagagg ggcgagggca 4260
   acagaccttt catgggcaaa ccttggggcc ctgctgtcct cctgtcacct ccagagccaa   4320
   gggatcaaag gaggaggagc caggacagga gggaagtggg agggagggtc ccagcagagg   4380
   actccaaatt taggcagcag gcatatggga tgggatataa aggggctgga gcactgagag   4440
   ctgtcagaga tttctccaac ccaggtaaga gggagtttcg ggtgggggct cttcacccac   4500
   accagacctc tccccaccta gaaggaaact gcctttcctg gaagtggggt tcaggccggt   4560
   cagagatctg acagggtggc cttccaccag cctgggaagt tctcagtggc aggaggtttc   4620
   cacaagaaac actggatgcc ccttccctta cgctgtcttc tccatcttcc tcctggggat   4680
   gctcctcccc gtcttggttt atcttggctc ttcgtcttca gcaagatttg ccctgtgctg   4740
   tccactccat ctttctctac tgtctccgtg ccttgccttg ccttcttgcg tgtccttcct   4800
   ttccacccat ttctcacttc accttttctc cccttctcat ttgtattcat ccttccttcc   4860
   ttccttcctt ccttccttcc ttccttcctt ccttcctttc tcccttcctt ccttccttcc   4920
   ttccttcctt ccttccttcc ttcctgtgtc agagtgctga gaatcacacc tggggttccc   4980
   acccttatgt aaacaatctt ccagtgagcc acagcttcag tgctgctggg tgctctctta   5040
   ccttcctcac cccctggctt gtcctgttcc atcctggtca ggatctctag attggtctcc   5100
   cagcctctgc tactcctctt cctgcctgtt cctctctctg tccagctgcg ccactgtggt   5160
   gcctcgttcc agctgtggtc cacattcttc aggattctct gaaaagttaa ccaggtgaga   5220
   atgtttcccc tgtagacagc agatcacgat tctcccggaa gtcaggcttc cagccctctc   5280
   tttctctgcc cagctgcccg gcactcttag caaacctcag gcacccttac cccacataga   5340
   cctctgacag agaagcaggc actttacatg gagtcctggt gggagagcca taggctacgg   5400
   tgtaaaagag gcagggaagt ggtggtgtag gaaagtcagg acttcacata gaagcctagc   5460
   ccacaccaga aatgacagac agatccctcc tatctccccc ataagagttt gagtcgaggg   5520
   atctaagcag aagcttcgaa tcgcgaattc gcccacc atg ctg ctg ctg ctg ctg    5575
                                           Met Leu Leu Leu Leu Leu
                                           1               5
   ctg ctg ggc ctg agg cta cag ctc tcc ctg ggc atc atc cca gtt gag     5623
   Leu Leu Gly Leu Arg Leu Gln Leu Ser Leu Gly Ile Ile Pro Val Glu
               10                  15                  20
   gag gag aac ccg gac ttc tgg aac cgc gag gca gcc gag gcc ctg ggt     5671
   Glu Glu Asn Pro Asp Phe Trp Asn Arg Glu Ala Ala Glu Ala Leu Gly
           25                  30                  35
   gcc gcc aag aag ctg cag cct gca cag aca gcc gcc aag aac ctc atc     5719
   Ala Ala Lys Lys Leu Gln Pro Ala Gln Thr Ala Ala Lys Asn Leu Ile
       40                  45                  50
   atc ttc ctg ggc gat ggg atg ggg gtg tct acg gtg aca gct gcc agg     5767
   Ile Phe Leu Gly Asp Gly Met Gly Val Ser Thr Val Thr Ala Ala Arg
   55                  60                  65                  70
   atc cta aaa ggg cag aag aag gac aaa ctg ggg cct gag ata ccc ctg     5815
   Ile Leu Lys Gly Gln Lys Lys Asp Lys Leu Gly Pro Glu Ile Pro Leu
                   75                  80                  85
   gcc atg gac cgc ttc cca tat gtg gct ctg tcc aag aca tac aat gta      5863
   Ala Met Asp Arg Phe Pro Tyr Val Ala Leu Ser Lys Thr Tyr Asn Val
               90                  95                  100
   gac aaa cat gtg cca gac agt gga gcc aca gcc acg gcc tac ctg tgc      5911
   Asp Lys His Val Pro Asp Ser Gly Ala Thr Ala Thr Ala Tyr Leu Cys
           105                 110                 115
   ggg gtc aag ggc aac ttc cag acc att ggc ttg agt gca gcc gcc cgc      5959
   Gly Val Lys Gly Asn Phe Gln Thr Ile Gly Leu Ser Ala Ala Ala Arg
       120                 125                 130
   ttt aac cag tgc aac acg aca cgc ggc aac gag gtc atc tcc gtg atg      6007
   Phe Asn Gln Cys Asn Thr Thr Arg Gly Asn Glu Val Ile Ser Val Met
   135                 140                 145                 150
   aat cgg gcc aag aaa gca ggg aag tca gtg gga gtg gta acc acc aca      6055
   Asn Arg Ala Lys Lys Ala Gly Lys Ser Val Gly Val Val Thr Thr Thr
                   155                 160                 165
   cga gtg cag cac gcc tcg cca gcc ggc acc tac gcc cac acg gtg aac      6103
   Arg Val Gln His Ala Ser Pro Ala Gly Thr Tyr Ala His Thr Val Asn
               170                 175                 180
   cgc aac tgg tac tcg gac gcc gac gtg cct gcc tcg gcc cgc cag gag      6151
   Arg Asn Trp Tyr Ser Asp Ala Asp Val Pro Ala Ser Ala Arg Gln Glu
           185                 190                 195
   ggg tgc cag gac atc gct acg cag ctc atc tcc aac atg gac att gac      6199
   Gly Cys Gln Asp Ile Ala Thr Gln Leu Ile Ser Asn Met Asp Ile Asp
       200                 205                 210
   gtg atc cta ggt gga ggc cga aag tac atg ttt cgc atg gga acc cca      6247
   Val Ile Leu Gly Gly Gly Arg Lys Tyr Met Phe Arg Met Gly Thr Pro
   215                 220                 225                 230
   gac cct gag tac cca gat gac tac agc caa ggt ggg acc agg ctg gac      6295
   Asp Pro Glu Tyr Pro Asp Asp Tyr Ser Gln Gly Gly Thr Arg Leu Asp
                   235                 240                 245
   ggg aag aat ctg gtg cag gaa tgg ctg gcg aag cgc cag ggt gcc cgg      6343
   Gly Lys Asn Leu Val Gln Glu Trp Leu Ala Lys Arg Gln Gly Ala Arg
               250                 255                 260
   tat gtg tgg aac cgc act gag ctc atg cag gct tcc ctg gac ccg tct      6391
   Tyr Val Trp Asn Arg Thr Glu Leu Met Gln Ala Ser Leu Asp Pro Ser
           265                 270                 275
   gtg acc cat ctc atg ggt ctc ttt gag cct gga gac atg aaa tac gag      6439
   Val Thr His Leu Met Gly Leu Phe Glu Pro Gly Asp Met Lys Tyr Glu
       280                 285                 290
   atc cac cga gac tcc aca ctg gac ccc tcc ctg atg gag atg aca gag      6487
   Ile His Arg Asp Ser Thr Leu Asp Pro Ser Leu Met Glu Met Thr Glu
   295                 300                 305                 310
   gct gcc ctg cgc ctg ctg agc agg aac ccc cgc ggc ttc ttc ctc ttc      6535
   Ala Ala Leu Arg Leu Leu Ser Arg Asn Pro Arg Gly Phe Phe Leu Phe
                   315                 320                 325
   gtg gag ggt ggt cgc atc gac cat ggt cat cat gaa agc agg gct tac      6583
   Val Glu Gly Gly Arg Ile Asp His Gly His His Glu Ser Arg Ala Tyr
               330                 335                 340
   cgg gca ctg act gag acg atc atg ttc gac gac gcc att gag agg gcg      6631
   Arg Ala Leu Thr Glu Thr Ile Met Phe Asp Asp Ala Ile Glu Arg Ala
           345                 350                 355
   ggc cag ctc acc agc gag gag gac acg ctg agc ctc gtc act gcc gac      6679
   Gly Gln Leu Thr Ser Glu Glu Asp Thr Leu Ser Leu Val Thr Ala Asp
       360                 365                 370
   cac tcc cac gtc ttc tcc ttc gga ggc tac ccc ctg cga ggg agc tcc      6727
   His Ser His Val Phe Ser Phe Gly Gly Tyr Pro Leu Arg Gly Ser Ser
   375                 380                 385                 390
   atc ttc ggg ctg gcc cct ggc aag gcc cgg gac agg aag gcc tac acg      6775
   Ile Phe Gly Leu Ala Pro Gly Lys Ala Arg Asp Arg Lys Ala Tyr Thr
                   395                 400                 405
   gtc ctc cta tac gga aac ggt cca ggc tat gtg ctc aag gac ggc gcc      6823
   Val Leu Leu Tyr Gly Asn Gly Pro Gly Tyr Val Leu Lys Asp Gly Ala
               410                 415                 420
   cgg ccg gat gtt acc gag agc gag agc ggg agc ccc gag tat cgg cag      6871
   Arg Pro Asp Val Thr Glu Ser Glu Ser Gly Ser Pro Glu Tyr Arg Gln
           425                 430                 435
   cag tca gca gtg ccc ctg gac gaa gag acc cac gca ggc gag gac gtg      6919
   Gln Ser Ala Val Pro Leu Asp Glu Glu Thr His Ala Gly Glu Asp Val
       440                 445                 450
   gcg gtg ttc gcg cgc ggc ccg cag gcg cac ctg gtt cac ggc gtg cag      6967
   Ala Val Phe Ala Arg Gly Pro Gln Ala His Leu Val His Gly Val Gln
   455                 460                 465                 470
   gag cag acc ttc ata gcg cac gtc atg gcc ttc gcc gcc tgc ctg gag      7015
   Glu Gln Thr Phe Ile Ala His Val Met Ala Phe Ala Ala Cys Leu Glu
                   475                 480                 485
   ccc tac acc gcc tgc gac ctg gcg ccc ccc gcc ggc acc acc gac gcc      7063
   Pro Tyr Thr Ala Cys Asp Leu Ala Pro Pro Ala Gly Thr Thr Asp Ala
               490                 495                 500
   gcg cac ccg ggt tac tct aga gtc ggg gcg gcc ggc cgc ttc gag cag      7111
   Ala His Pro Gly Tyr Ser Arg Val Gly Ala Ala Gly Arg Phe Glu Gln
           505                 510                 515
   aca tga taagatacat tgatgagttt ggacgcggcc gcgactctag atcataatca       7167
   Thr
   gccataccac atttgtagag gttttacttg ctttaaaaaa cctcccacac ctccccctga    7227
   acctgaaaca taaaatgaat gcaattgttg ttgttaactt gtttattgca gcttataatg    7287
   gttacaaata aagcaatagc atcacaaatt tcacaaataa agcatttttt tcactgcatt    7347
   ctagttgtgg tttgtccaaa ctcatcaatg tatcttaagg cgtaaattgt aagcgttaat    7407
   attttgttaa aattcgcgtt aaatttttgt taaatcagct cattttttaa ccaataggcc    7467
   gaaatcggca aaatccctta taaatcaaaa gaatagaccg agatagggtt gagtgttgtt    7527
   ccagtttgga acaagagtcc actattaaag aacgtggact ccaacgtcaa agggcgaaaa 7587
   accgtctatc agggcgatgg cccactacgt gaaccatcac cctaatcaag ttttttgggg 7647
   tcgaggtgcc gtaaagcact aaatcggaac cctaaaggga gcccccgatt tagagcttga 7707
   cggggaaagc cggcgaacgt ggcgagaaag gaagggaaga aagcgaaagg agcgggcgct 7767
   agggcgctgg caagtgtagc ggtcacgctg cgcgtaacca ccacacccgc cgcgcttaat 7827
   gcgccgctac agggcgcgtc aggtggcact tttcggggaa atgtgcgcgg aacccctatt 7887
   tgtttatttt tctaaataca ttcaaatatg tatccgctca tgagacaata accctgataa 7947
   atgcttcaat aatattgaaa aaggaagagt cctgaggcgg aaagaaccag ctgtggaatg 8007
   tgtgtcagtt agggtgtgga aagtccccag gctccccagc aggcagaagt atgcaaagca 8067
   tgcatctcaa ttagtcagca accaggtgtg gaaagtcccc aggctcccca gcaggcagaa 8127
   gtatgcaaag catgcatctc aattagtcag caaccatagt cccgccccta actccgccca 8187
   tcccgcccct aactccgccc agttccgccc attctccgcc ccatggctga ctaatttttt 8247
   ttatttatgc agaggccgag gccgcctcgg cctctgagct attccagaag tagtgaggag 8307
   gcttttttgg aggcctaggc ttttgcaaag atcgatcaag agacaggatg aggatcgttt 8367
   cgcatgattg aacaagatgg attgcacgca ggttctccgg ccgcttgggt ggagaggcta 8427
   ttcggctatg actgggcaca acagacaatc ggctgctctg atgccgccgt gttccggctg 8487
   tcagcgcagg ggcgcccggt tctttttgtc aagaccgacc tgtccggtgc cctgaatgaa 8547
   ctgcaagacg aggcagcgcg gctatcgtgg ctggccacga cgggcgttcc ttgcgcagct 8607
   gtgctcgacg ttgtcactga agcgggaagg gactggctgc tattgggcga agtgccgggg 8667
   caggatctcc tgtcatctca ccttgctcct gccgagaaag tatccatcat ggctgatgca 8727
   atgcggcggc tgcatacgct tgatccggct acctgcccat tcgaccacca agcgaaacat 8787
   cgcatcgagc gagcacgtac tcggatggaa gccggtcttg tcgatcagga tgatctggac 8847
   gaagagcatc aggggctcgc gccagccgaa ctgttcgcca ggctcaaggc gagcatgccc 8907
   gacggcgagg atctcgtcgt gacccatggc gatgcctgct tgccgaatat catggtggaa 8967
   aatggccgct tttctggatt catcgactgt ggccggctgg gtgtggcgga ccgctatcag 9027
   gacatagcgt tggctacccg tgatattgct gaagagcttg gcggcgaatg ggctgaccgc 9087
   ttcctcgtgc tttacggtat cgccgctccc gattcgcagc gcatcgcctt ctatcgcctt 9147
   cttgacgagt tcttctgagc gggactctgg ggttcgaaat gaccgaccaa gcgacgccca 9207
   acctgccatc acgagatttc gattccaccg ccgccttcta tgaaaggttg ggcttcggaa 9267
   tcgttttccg ggacgccggc tggatgatcc tccagcgcgg ggatctcatg ctggagttct 9327
   tcgcccaccc taggggaggc ctaactgaaa cacggaagga gacaataccg gaaggaaccc 9387
   gcgctatgac ggcaataaaa agacagaata aaacgcacgg tgttgggtcg tttgttcata    9447
   aacgcggggt tcggtcccag ggctggcact ctgtcgatac cccaccgaga ccccattggg    9507
   gccaatacgc ccgcgtttct tccttttccc caccccaccc cccaagttcg ggtgaaggcc    9567
   cagggctcgc agccaacgtc ggggcggcag gccctgccat agcctcaggt tactcatata    9627
   tactttagat tgatttaaaa cttcattttt aatttaaaag gatctaggtg aagatccttt    9687
   ttgataatct catgaccaaa atcccttaac gtgagttttc gttccactga gcgtcagacc    9747
   ccgtagaaaa gatcaaagga tcttcttgag atcctttttt tctgcgcgta atctgctgct    9807
   tgcaaacaaa aaaaccaccg ctaccagcgg tggtttgttt gccggatcaa gagctaccaa    9867
   ctctttttcc gaaggtaact ggcttcagca gagcgcagat accaaatact gtccttctag    9927
   tgtagccgta gttaggccac cacttcaaga actctgtagc accgcctaca tacctcgctc    9987
   tgctaatcct gttaccagtg gctgctgcca gtggcgataa gtcgtgtctt accgggttgg   10047
   actcaagacg atagttaccg gataaggcgc agcggtcggg ctgaacgggg ggttcgtgca   10107
   cacagcccag cttggagcga acgacctaca ccgaactgag atacctacag cgtgagctat   10167
   gagaaagcgc cacgcttccc gaagggagaa aggcggacag gtatccggta agcggcaggg   10227
   tcggaacagg agagcgcacg agggagcttc cagggggaaa cgcctggtat ctttatagtc   10287
   ctgtcgggtt tcgccacctc tgacttgagc gtcgattttt gtgatgctcg tcaggggggc   10347
   ggagcctatg gaaaaacgcc agcaacgcgg cctttttacg gttcctggcc ttttgctggc   10407
   cttttgctca catgttcttt cctgcgttat cccctgattc tgtggataac cgtattaccg   10467
   ccatgcat                                                            10475
<210> 4
   <211> 519
   <212> PRT
   <213> SEAP
<400> 4
   Met Leu Leu Leu Leu Leu Leu Leu Gly Leu Arg Leu Gln Leu Ser Leu
   1               5                   10                  15
   Gly Ile Ile Pro Val Glu Glu Glu Asn Pro Asp Phe Trp Asn Arg Glu
               20                  25                  30
   Ala Ala Glu Ala Leu Gly Ala Ala Lys Lys Leu Gln Pro Ala Gln Thr
           35                  40                  45
   Ala Ala Lys Asn Leu Ile Ile Phe Leu Gly Asp Gly Met Gly Val Ser
       50                  55                  60
   Thr Val Thr Ala Ala Arg Ile Leu Lys Gly Gln Lys Lys Asp Lys Leu
   65                  70                  75                  80
   Gly Pro Glu Ile Pro Leu Ala Met Asp Arg Phe Pro Tyr Val Ala Leu
                   85                  90                  95
   Ser Lys Thr Tyr Asn Val Asp Lys His Val Pro Asp Ser Gly Ala Thr
               100                 105                 110
   Ala Thr Ala Tyr Leu Cys Gly Val Lys Gly Asn Phe Gln Thr Ile Gly
           115                 120                 125
   Leu Ser Ala Ala Ala Arg Phe Asn Gln Cys Asn Thr Thr Arg Gly Asn
       130                 135                 140
   Glu Val Ile Ser Val Met Asn Arg Ala Lys Lys Ala Gly Lys Ser Val
   145                 150                 155                 160
   Gly Val Val Thr Thr Thr Arg Val Gln His Ala Ser Pro Ala Gly Thr
                   165                 170                 175
   Tyr Ala His Thr Val Asn Arg Asn Trp Tyr Ser Asp Ala Asp Val Pro
               180                 185                 190
   Ala Ser Ala Arg Gln Glu Gly Cys Gln Asp Ile Ala Thr Gln Leu Ile
           195                 200                 205
   Ser Asn Met Asp Ile Asp Val Ile Leu Gly Gly Gly Arg Lys Tyr Met
       210                 215                 220
   Phe Arg Met Gly Thr Pro Asp Pro Glu Tyr Pro Asp Asp Tyr Ser Gln
   225                 230                 235                 240
   Gly Gly Thr Arg Leu Asp Gly Lys Asn Leu Val Gln Glu Trp Leu Ala
                   245                 250                 255
   Lys Arg Gln Gly Ala Arg Tyr Val Trp Asn Arg Thr Glu Leu Met Gln
               260                 265                 270
   Ala Ser Leu Asp Pro Ser Val Thr His Leu Met Gly Leu Phe Glu Pro
           275                 280                 285
   Gly Asp Met Lys Tyr Glu Ile His Arg Asp Ser Thr Leu Asp Pro Ser
       290                 295                 300
   Leu Met Glu Met Thr Glu Ala Ala Leu Arg Leu Leu Ser Arg Asn Pro
   305                 310                 315                 320
   Arg Gly Phe Phe Leu Phe Val Glu Gly Gly Arg Ile Asp His Gly His
                   325                 330                 335
   His Glu Ser Arg Ala Tyr Arg Ala Leu Thr Glu Thr Ile Met Phe Asp
               340                 345                 350
   Asp Ala Ile Glu Arg Ala Gly Gln Leu Thr Ser Glu Glu Asp Thr Leu
           355                 360                 365
   Ser Leu Val Thr Ala Asp His Ser His Val Phe Ser Phe Gly Gly Tyr
       370                 375                 380
   Pro Leu Arg Gly Ser Ser Ile Phe Gly Leu Ala Pro Gly Lys Ala Arg
   385                 390                 395                 400
   Asp Arg Lys Ala Tyr Thr Val Leu Leu Tyr Gly Asn Gly Pro Gly Tyr
                   405                 410                 415
   Val Leu Lys Asp Gly Ala Arg Pro Asp Val Thr Glu Ser Glu Ser Gly
               420                 425                 430
   Ser Pro Glu Tyr Arg Gln Gln Ser Ala Val Pro Leu Asp Glu Glu Thr
           435                 440                 445
   His Ala Gly Glu Asp Val Ala Val Phe Ala Arg Gly Pro Gln Ala His
       450                 455                 460
   Leu Val His Gly Val Gln Glu Gln Thr Phe Ile Ala His Val Met Ala
   465                 470                 475                 480
   Phe Ala Ala Cys Leu Glu Pro Tyr Thr Ala Cys Asp Leu Ala Pro Pro
                   485                 490                 495
   Ala Gly Thr Thr Asp Ala Ala His Pro Gly Tyr Ser Arg Val Gly Ala
               500                 505                 510
   Ala Gly Arg Phe Glu Gln Thr
           515

## Description

### Field of the invention

The present invention relates to the use of proteins capable of being secreted across the cell membrane as cell type-specific reporters during cell development. In detail, the present invention relates to embryonic stem (ES) cells stably transfected with a DNA construct comprising a DNA sequence encoding a secreted protein, which is operably linked to a cell-and/or development-dependent promoter; a method for preparing such ES cells; a cell culture obtainable by culturing said ES cells; a method for the toxicological examination of substances using such cell cultures as well as to methods for the identification of differentiation and growth factors for induction of selectively differentiated cells which can be used as a source for tissue grafts; a method for producing transgenic non-human mammals using said ES cells; a transgenic non-human mammal obtainable by said method; and to a method for examining stages of cellular development using cells of such a non-human mammal. The present invention also concerns assays to identify culture conditions and substances that promote the differentiation into a desired cell type, or to determine the toxicity of a compound for certain cell types. Those assays are particularly suited to give indications of possible developmental and differentiation disturbances during embryogenesis.

### Background art

Precursor cells have become a central interest in medical research. On the one hand precursors can replace cells that are senescent or damaged by injury or disease and on the other hand these cells represent an ideal model for studying development and differentiation and the factors influencing these processes. Employing conventional cell lines for these studies has the disadvantage that individual cell lines may not.be fully representative of the complex biology of an intact organism. Moreover, even repeating the tests in multiple cell lines does not reproduce or account for the complex interactions among cells and tissue that occur in an organism.

This is the main reason why toxicity testing in cell lines can not replace conventional animal tests. New chemical compounds are constantly being developed and tested on animals. In addition to industrial and household chemicals, a number of chemical compositions are developed each year for use as pharmaceuticals. Rules regarding the testing of potential pharmaceuticals are promulgated by the Food and Drug Administration ("FDA"), which currently requires comprehensive testing of toxicity, mutagenicity, and other effects in at least two species before a drug candidate can be entered into human clinical trials. Preclinical toxicity testing alone costs some hundreds of thousands of dollars. Despite this huge investment, almost one third of all prospective human therapeutics fail in the first phase of human clinical trials because of unexpected toxicity. It is clear that currently available toxicological screening assays do not detect all toxicities associated with human therapy or exposure to chemicals in the environment. Better means of screening potential therapeutics or chemicals in general for potential toxicity would reduce the cost and uncertainty of developing new therapeutics and materials, for example for use in medical devices or in other devices or goods humans are exposed to every day.

The detection of teratogenic and/or embryotoxic properties of chemical agents occurs presently by determination of the reproduction toxicity of test substances following single or multi-administrations to pregnant laboratory mammals and by tests of the embryotoxicity in the early stages of pregnancy. Furthermore, in vitro tests are performed with mammal embryos (Neubert and Merker, de Gruyter, Berlin-New York (1981)) and with embryonic organs for teratogenicity tests. These test procedures have however the disadvantage that they require the use of a large number of live mammals, in particular rats and mice. In vitro test procedures, in which primary cell cultures of limb buds (for example, "Limb Buds", Kochhar, Teratology 11 (1975), 273-287), or brain parts of embryonic rats (Flint and Orton, Toxicol. Appl. Pharmacol. 76 (1984), 383-395) or permanent cell lines of embryonic or adult mammal tissue such as tumor cells of the ovary or embryonic palate cells are employed, do not fulfill, strictly speaking, the requirements which are imposed on the teratogenicity tests during the embryogenesis, namely giving indications of possible disgenesis or developmental disturbances.

Efforts have been made for a couple of years to employ permanent cultures of totipotent/pluripotent embryonic stem cells (ES cells) for the detection of embryotoxic and mutagenic substances; see, e.g. Laschinski et al., Reproductive Toxicol. 5 (1991), 57-65; Newall and Beedles, Toxicol. in Vitro 8 (1994), 697-701; Sehlmeyer and Wobus, Mutation Res. 324 (1994), 69-76.

All those systems make use of the biological properties of the test ES cells or of a reporter system that has been introduced into the cells.

For example, US-A-6,498,018 describes a method for determining the effect of a biological agent by contacting a cell culture with a biological agent. The cell culture contains human multipotent CNS neural stem cells that are derived from primary CNS neural tissue and a culture medium with pre-selected growth factors. The read-out is provided by the effect of a biological agent on the presence or absence of a biological function or property ascribable to the cell culture. A major disadvantage of this system is the fact that particular biological functions or properties inherent to a certain culture of cells are difficult to measure and often involve the destruction of a large part of the culture in order to obtain enough material for the assay.

WO02/086073 discloses a method for the positive selection of neuronal cells differentiated from nuclear transfer embryonic stem cells by taking advantage of the neural stem cell marker nectin. This method is limited to neural cell types expressing nectin naturally. US-A-6,007,993 describes an in vitro test procedure for the detection of chemically-induced embryotoxic (for example also teratogenic) effects based on differentiated pluripotent embryonic stem (ES) cells from the mouse and rat and using embryonic germ (EG) cells established from primordial germ cells. Stable transgenic ES or EG stem cell clones are constructed, wherein a bacterial reporter gene, LacZ or the luciferase gene, is brought under the control of tissue-specific promoters or developmental control genes. Following differentiation of the ES cells in the presence of teratogenic substances into the different germination path derivatives, there occurs a differentiation-dependent expression in the cells, due to the activity of the tissue-specific promoters. The activation, repression or modulation of these tissue-specific genes is detected based on a reaction depending on the reporter gene employed, for example the X-Gal assay.

Wobus et al., J. Mol. Cell. Cardiol. 29 (1997), 1525-1539 described a method for the monitoring of cell differentiation including culturing embryonic stem (ES) cells capable of differentiating and forming embryoid bodies, which contain a recombinant nucleic acid 5 molecule comprising a reporter gene encoding the lacZ gene under the control of MLC-2v promoter region, wherein the amount or activity of the lacZ gene product is used for monitoring the differentiation of the cells in the presence of a retinoic acid.

WO99/01552 discloses embryonic stem (ES) cells, which are transfected in a stable manner with a DNA construct encoding a non-cell-damaging fluorescent protein and operatively linked thereto a cell- or development-dependent promoter. Also disclosed is a method for toxicological monitoring of substances using these ES cell cultures.

Although the above described methods employ semi-quantitative as well as relatively simple and robust assays, those assays usually involve the destruction of the tested cells. The preparation of a cell lysate required by many of the described conventional assay systems results in a high intra- as well as inter-assay variability. For repeated measurements during the course of differentiation many parallel cultures have to be assayed with the associated problems of variability. Laborious preparation of lysates also renders the assay less amenable to high-throughput formats. Using fluorescent reporter proteins can overcome some of those problems. However, GFP expression has already shown some toxic effects (Liu et al. Biochem. Biophys. Res. Comm. 260 (1999), 712-717). Another disadvantage is the autofluorescence of aggregates of ES cells (embryoid bodies) which gives a high background in fluorescence measurements.

The technical problem of readily monitoring cells differentiating into a certain cell type and thereby providing a reliable and highly sensitive test system for toxicity screening, differentiation promoting agents and/or appropriate culture conditions, medium supplements, is achieved by providing the embodiments characterized in the claims, and described further below.

### Summary of the invention

The present invention relates to a method of monitoring cell differentiation comprising culturing cells capable of differentiating into at least one particular cell type, wherein said cells contain at least one recombinant nucleic acid molecule comprising a reporter gene encoding a product that is secreted upon cell differentiation, under conditions allowing differentiation of the cells, or maintaining a non-human animal comprising such cells under appropriate conditions; and determining the amount of activity or amount of the reporter gene product either within a body fluid of said transgenic non-human animal or the cell culture medium or sample thereof.

The present invention also concerns the mentioned reporter gene constructs for monitoring cell differentiation, as well as cells, aggregates of at least one cell type, tissue comprising those cells or aggregates, organs comprising said cells or tissue, implants and transplants comprising said organ, all of which are characterized by comprising such a reporter gene construct.

Furthermore, the present invention relates to a non-human animal and a composition of matter both characterized by comprising a reporter gene construct, a cell, a cell aggregate, a tissue and/or an organ described herein.

In addition, the instant invention relates to an array or chip which contains the cells, cell aggregates or tissue of the invention attached to a solid support or suspended thereon as well as to an apparatus for analyzing said array or chip.

In particular, the present invention relates to test systems to identify substances that influence the differentiation of cells into certain cell types. Therefore, the present invention provides a method for obtaining and/or profiling a modulator of cell differentiation. This method comprises contacting a test sample containing a cell, cell aggregate, tissue, organ or a non-human transgenic animal as defined above with the substance to be tested; and then determining the effect of the test substance on the amount of the reporter gene product or activity compared to a control sample or animal.

The test system provided by the present invention is useful for drug screening purposes. It is envisaged to obtain and manufacture drugs which promote or inhibit the formation of said specific cell types using the method described above, wherein an enhanced or reduced level or activity of the reporter gene product is indicative for the drug. An enhanced or reduced level or activity of the reporter gene product, respectively, can also be indicative for an agent or compound which supports wound healing and/or healing of damaged tissue or toxicity of compounds, respectively.

The present invention additionally relates to a kit useful for conducting the methods described above, which contains, for example, a reporter gene construct and/or a cell as defined above, and standard compounds, like cell culture media, selection agents, detection agents for the reporter molecule and control samples.

Furthermore, methods of conducting a drug discovery and/or a target discovery business are disclosed. Both methods involve providing one or more assay systems described above for the identification of modulators of cell differentiation; and/or conducting therapeutic profiling of the modulators identified, or further analogs thereof, for efficacy and toxicity in the transgenic animals provided by the present invention. The modulators identified using the methods provided by the invention are then either formulated in a pharmaceutical composition or licensed to a third party for further drug development and/or sales.

The present invention also relates to modulators of cell differentiation which have been identified using the methods of the invention as well as to pharmaceutical compositions comprising such modulators for use in the modulation of cell differentiation and the treatment of corresponding diseases.

Furthermore, the present invention relates to the use of a reporter gene construct, a cell, a cell aggregate, tissue, organ, implant or transplant, a non-human animal, a composition, an array or an apparatus provided herein in drug discovery, pharmacokinetic or pharmacological profiling.

### Brief description of the figure

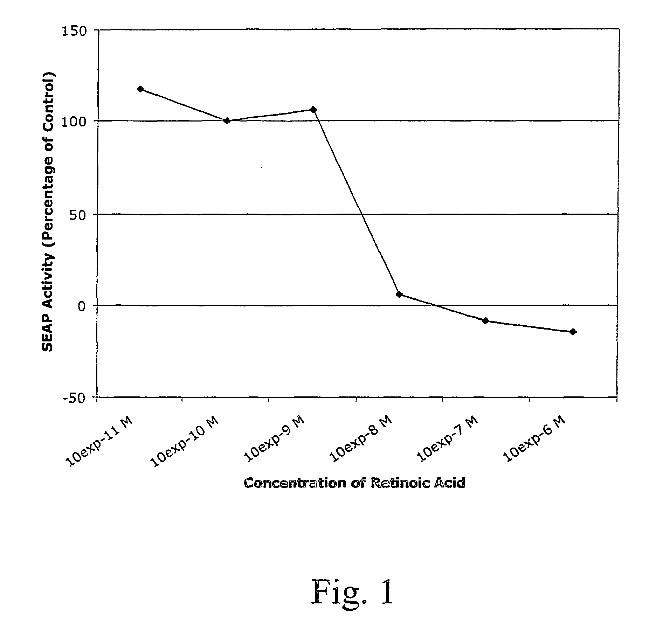

- **Figure 1:**: SEAP expression driven by the cardiac-specific αMHC promoter by cells differentiated from ES cells in the presence of retinoic acid. ES cells transfected with the α-MHC-SEAP vector (containing the SEAP gene under the control of the heart-specific αMHC promoter) were differentiated in the presence of all-*trans* retinoic acid at concentrations indicated. After 13 days culture supernatants were assayed for SEAP activity. Shown are mean values of duplicate samples from which background SEAP activity was substracted. Activities are expressed as percentage of SEAP activity in samples containing only solvent (DMSO) but no retinoic acid.

### Definitions

For the purposes of this description, the term "stem cell" can refer to either stem cell or germ cell, for example embryonic stem (ES) and germ (EG) cell, respectively. Minimally, a stem cell has the ability to proliferate and form cells of more than one different phenotype, and is also capable of self-renewal, either as part of the same culture, or when cultured under different conditions. Embryonic stem cells are also typically telomerase-positive and OCT-4 positive. Telomerase activity can be determined using TRAP activity assay (Kim et al., Science 266 (1997), 2011), using a commercially available kit (TRAPeze(R) XK Telomerase Detection Kit, Cat. s7707; Intergen Co., Purchase N.Y.; or TeloTAGGG(TM) Telomerase PCR ELISAplus, Cat. 2,013,89; Roche Diagnostics, Indianapolis). hTERT expression can also be evaluated at the mRNA level by RT-PCR. The LightCycler TeloTAGGG(TM) hTERT quantification kit (Cat. 3,012,344; Roche Diagnostics) is available commercially for research purposes.

In accordance with the present invention, the term embryonic stem (ES) cell includes any multi- or pluripotent stem cell derived from pre-embryonic, embryonic or fetal tissue at any time after fertilization, and having the characteristic of being capable under appropriate conditions of producing progeny of several different cell types that are derivatives of all of the three germinal layers (endoderm, mesoderm and ectoderm), according to a standard art-accepted test, such as the ability to form a teratoma in 8-12 week old SCID mice.

"Embryonic germ cells" or "EG cells" are cells derived from primordial germ cells. The term "embryonic germ cell" is used to describe cells of the present invention that exhibit an embryonic pluripotent cell phenotype. The terms "human embryonic germ cell (EG)" or "embryonic germ cell" can be used interchangeably herein to describe mammalian, preferably human cells, or cell lines thereof, of the present invention that exhibit a pluripotent embryonic stem cell phenotype as defined herein. Thus, EG cells are capable of differentiation into cells of ectodermal, endodermal and mesodermal germ layers. EG cells can also be characterized by the presence or absence of markers associated with specific epitope sites identified by the binding of particular antibodies and the absence of certain markers as identified by the lack of binding of certain antibodies.

"Pluripotent" refers to cells that retain the developmental potential to differentiate into a wide range of cell lineages including the germ line. The terms "embryonic stem cell phenotype" and "embryonic stem-like cell" also are used interchangeably herein to describe cells that are undifferentiated and thus are pluripotent cells.

Included in the definition of ES cells are embryonic cells of various types, exemplified by human embryonic stem cells, described by Thomson et al. (Science 282 (1998), 1145); embryonic stem cells from other primates, such as rhesus stem cells (Thomson et al., Proc. Natl. Acad. Sci. USA 92 (1995), 7844), marmoset stem cells (Thomson et al., Biol. Reprod. 55 (1996), 254) and human embryonic germ (hEG) cells (Shamblott et al., Proc. Natl. Acad. Sci. USA 95 (1998), 13726). Other types of pluripotent cells are also included in the term. Any cells of mammalian origin that are capable of producing progeny that are derivatives of all three germinal layers are included, regardless of whether they were derived from embryonic tissue, fetal tissue, or other sources. The stem cells employed in accordance with the present invention are preferably (but not always necessarily) karyotypically normal. However, it is preferred not to use ES cells that are derived from a malignant source.

"Feeder cells" or "feeders" are terms used to describe cells of one type that are co-cultured with cells of another type, to provide an environment in which the cells of the second type can grow. The feeder cells are optionally from a different species as the cells they are supporting. For example, certain types of ES cells can be supported by primary mouse embryonic fibroblasts, immortalized mouse embryonic fibroblasts (such as murine STO cells, e.g., Martin and Evans, Proc. Natl. Acad. Sci. USA 72 (1975), 1441-1445), or human fibroblast-like cells differentiated from human ES cells, as described later in this disclosure. The term "STO cell" refers to embryonic fibroblast mouse cells such as are commercially available and include those deposited as ATCC CRL 1503.

The term "embryoid bodies" (EBs) is a term of art synonymous with "aggregate bodies". The terms refer to aggregates of differentiated and undifferentiated cells that appear when ES cells overgrow in monolayer cultures, or are maintained in suspension cultures. Embryoid bodies are a mixture of different cell types, typically from several germ layers, distinguishable by morphological criteria; see also infra. As used herein, "embryoid body", "EB"or "EB cells" typically refers to a morphological structure comprised of a population of cells, the majority of which are derived from embryonic stem (ES) cells that have undergone differentiation. Under culture conditions suitable for EB formation (e.g., the removal of Leukemia inhibitory factor or other, similar blocking factors), ES cells proliferate and form small masses of cells that begin to differentiate. In the first phase of differentiation, usually corresponding to about days 1-4 of differentiation for humans, the small mass of cells forms a layer of endodermal cells on the outer layer, and is considered a "simple embryoid body". In the second phase, usually corresponding to about days 3-20 post differentiation for humans, "complex embryoid bodies" are formed, which are characterized by extensive differentiation of ectodermal and mesodermal cells and derivative tissue. As used herein, the term "embryoid body" or "EB" encompasses both simple and complex embryoid bodies unless otherwise required by context. The determination of when embryoid bodies have formed in a culture of ES cells is routinely made by persons of skill in the art by, for example, visual inspection of the morphology. Floating masses of about 20 cells or more are considered to be embryoid bodies; see, e.g., Schmitt et al., Genes Dev. 5 (1991), 728-740; Doetschman et al., J. Embryol. Exp. Morph. 87 (1985), 27-45. It is also understood that the term "embryoid body", "EB", or "EB cells" as used herein encompasses a population of cells, the majority of which being pluripotent cells capable of developing into different cellular lineages when cultured under appropriate conditions. As used herein, the term also refers to equivalent structures derived from primordial germ cells, which are primitive cells extracted from embryonic gonadal regions; see, e.g., Shamblott et al., (1998), supra. Primordial germ cells, sometimes also referred to in the art as EG cells or embryonic germ cells, when treated with appropriate factors form pluripotent ES cells from which embryoid bodies can be derived; see, e.g., US Patent US-A-5,670,372; Shamblott et al., supra.

The terms "polynucleotide" and "nucleic acid molecule" refer to a polymer of nucleotides of any length. Included are genes and gene fragments, mRNA, tRNA, rRNA, ribozymes, cDNA, recombinant polynucleotides, branched polynucleotides, plasmids, vectors, isolated DNA and RNA, nucleic acid probes, and primers. As used in this disclosure, the term polynucleotides refer interchangeably to double- and single-stranded molecules. Unless otherwise specified or required, any embodiment of the invention that is a polynucleotide encompasses both a double-stranded form, and each of the two complementary single-stranded forms known or predicted to make up the double-stranded form. Included are nucleic acid analogs such as phosporamidates and thiophosporamidates.

A cell is said to be "genetically altered", "transfected" or "genetically transformed" when a polynucleotide has been transferred into the cell by any suitable means of artificial manipulation, or where the cell is a progeny of the originally altered cell that has inherited the polynucleotide. The polynucleotide will often comprise a transcribable sequence encoding a protein of interest, which enables the cell to express the protein at an elevated level. The genetic alteration is said to be "inheritable" if progeny of the altered cell have the same alteration.

A "regulatory sequence" or "control sequence" is a nucleotide sequence involved in an interaction of molecules that contributes to the functional regulation of a polynucleotide, such as replication, duplication, transcription, splicing, polyadenylation, translation, or degradation of the polynucleotide. Transcriptional control elements include promoters, enhancers, and repressors.

Particular gene sequences referred to as promoters, like the "αMHC" or "collagen" promoter, are polynucleotide sequences derived from the gene referred to that promote transcription of an operatively linked gene expression product. It is recognized that various portions of the upstream and intron untranslated gene sequence may in some instances contribute to promoter activity, and that all or any subset of these portions may be present in the genetically engineered construct referred to. The promoter may be based on the gene sequence of any species having the gene, unless explicitly restricted, and may incorporate any additions, substitutions or deletions desirable, as long as the ability to promote transcription in the target tissue. Genetic constructs designed for treatment of humans typically comprise a segment that is at least 90 % identical to a promoter sequence of a human gene. A particular sequence can be tested for activity and specificity, for example, by operatively linking to a reporter gene; see Figure 1. -

According to the present invention, the term "cell- and/or development-dependent promoter" is intended to mean a promoter which displays its promoter activity only in particular cell types and/or only in particular stages of cellular development, both in cell cultures (embryoid bodies) and in transgenic non-human mammals derived from the ES cells according to the invention. In addition, any other known cell-specific promoter can be employed, e.g. for nerve cells, heart cells, neurons, glia cells, hematopoietic cells, endothelial cells, smooth muscle cells, skeletal muscle cells, cartilage cells, fibroblasts and epithelial cells.

Genetic elements are said to be "operatively linked" if they are in a structural relationship permitting them to operate in a manner according to their expected function. For instance, if a promoter helps to initiate transcription of the coding sequence, the coding sequence can be referred to as operatively linked to (or under control of) the promoter. There may be an intervening sequence between the promoter and coding region as long as this functional relationship is maintained.

In the context of encoding sequences, promoters and other genetic elements, the term "heterologous" indicates that the element is derived from a genotypically distinct entity from that of the rest of the entity to which it is being compared. For example, a promoter or gene introduced by genetic engineering techniques into an animal of a different species is said to be a heterologous polynucleotide. An "endogenous" genetic element is an element that is in the same place in the chromosome where it occurs in nature, although other elements may be artificially introduced into a neighboring position.

The terms "polypeptide", "peptide" and "protein" are used interchangeably in this disclosure to refer to polymers of amino acids of any length. The polymer may comprise modified amino acids, it may be linear or branched, and it may be interrupted by non-amino acids.

If not stated otherwise, the terms "compound", "substance" and "(chemical) composition" are used interchangeably herein and include, but are not limited to therapeutic agents (or potential therapeutic agents), agents of known toxicities such as neurotoxins, hepatic toxins, toxins of hematopoietic cells, myotoxins, carcinogens, teratogens, or toxins to one or more reproductive organs. The chemical compositions can further be agricultural chemicals, such as pesticides, fungicides, nematicides, and fertilizers, cosmetics, including so-called "cosmeceuticals", industrial wastes or by-products, or environmental contaminants. They can also be animal therapeutics or potential animal therapeutics.

Industrial products that can be tested with the methods of the present invention include bleaches, toilet, blocks, washing-up liquids, soap powders and liquids, fabric conditioners, window, oven, floor, bathroom, kitchen and carpet cleaners, dishwater detergents and rinse aids, water-softening agents, descalers, stain removers, polishes, oil products, paints, paint removers, glues, solvents, varnishes, air fresheners, moth balls and insecticides.

New ingredients for household products are constantly being developed and need to be tested. For example, in recent years new enzymes (to digest stains) and "optical brighteners" (which make washing appear whiter) have been developed for use in washing powders and liquids. New surfactants (which cut through grease to remove ingrained dirt) and chemical "builders" (which act as water softeners and enable surfactants to work more effectively) have been developed for use in washing powders and liquids, washing-up liquids and various cleaning agents. But also medical materials have to be tested, for example dental materials such as new filling polymers, metal alloys, and bioactive ceramic. Furthermore, chemical compositions of any part of a device, such as catheters, electrodes, adhesives, paste, gel or cream may be tested with the method of the present invention in different concentrations and with different ingredients and impurities present.

As used herein, "molecular profile" or "profile" of a chemical composition or compound refers to a pattern of alterations in gene or protein expression, or both, in an ES cell, embryoid body, tissue, etc. contacted by the chemical composition compared to a like cell, embryoid body or tissue in contact only with culture medium.

### Detailed description

Stem cells of various kinds have become an extremely attractive modality in regenerative medicine. They can be proliferated in culture, and then differentiated in vitro or in situ into the cell types needed. This plasticity makes them ideal models for toxicity testing as well as investigation of pharmaceuticals such as tissue-specific promoting factors. Particularly, embryoid bodies which consist of different cell types of the three germ layers that interact with each other provide a highly sensitive test system. The ability of the stem cells to differentiate into the different cell types in the presence of a test substance may depend on the ability of the substance to promote differentiation and/or the toxicity of said substance.

The method of monitoring cell differentiation in accordance with the present invention comprises:
- transfecting cells capable of differentiating into at least one particular cell type with at least one recombinant nucleic acid molecule comprising a reporter gene encoding a product that is secreted upon cell differentiation;
- introducing the cells into a non-human animal or culturing the cells under conditions allowing differentiation of the cells; and
- determining the amount or activity of the reporter gene product either within a body fluid of said transgenic non-human animal or the cell culture medium.

The present invention is based on the use of a reporter gene that is secreted. Surprisingly, it was found that secreted reporter molecules have certain advantages over the use of, for example, a fluorescent reporter like green fluorescent protein (GFP) and its derivatives. For example, Liu et al. (Biochem. Biophys. Res. Comm. 260 (1999) 712-717) observed toxic effects when using a GFP reporter. Such toxic effects can be avoided or at least minimized if a secreted reporter molecule is used, simply by exchanging the culturing medium on a regular basis and thereby preventing toxic concentration of the respective reporter molecule building up.

Also, a preferred method for the differentiation of cells is to grow them as embryoid bodies (EBs), which shows a high autofluorescence rate even when the cells were lysed. This background can render the use of fluorescent reporter genes for the monitoring of cell differentiation quite inefficient.

Other routinely used reporter molecules like, for example, beta-galactosidase or luciferase require the cells to be tested in a certain assay buffer which means that the cells have to be destroyed to determine enzyme activity. The system according to the invention avoids this by employing a secreted reporter molecule so that assaying the culture medium of the differentiating cells or the body fluid of an animal containing said cells is sufficient to monitor the reporter activity and thereby the differentiation.

This means, as shown in Example 3, that with the reporter system of the invention on the one hand it is possible to quantify stem cell-derived cell types such as cardiomyocytes and on the other hand or in addition to monitor the differentiation process of the individual stem cell. Accordingly, monitoring cell differentiation in accordance with the present invention includes both, i.e. monitoring cell differentiation by quantifying the differentiated cells and/or following the differentiation process of one or more of the corresponding undifferentiated cells, for example stem cells.

For the transfection of the nucleic acid molecule encoding the reporter protein any standard method known to the person skilled in the art can be employed. Any suitable expression vector for this purpose can be used. Suitable viral vector systems for producing stem cells altered according to this invention can be prepared using commercially available virus components. The introduction of the vector construct or constructs into the cells occurs in a known manner, e.g. by transfection or transduction with the help of viral vectors. Transfection of vector plasmids into cells can be accomplished by, e.g., electroporation or lipofection, i.e. using lipid/DNA complexes. Exemplary is the formulation Lipofectamine 2000(TM), available from Gibco/Life Technologies. Another exemplary reagent is FuGENE(TM) 6 Transfection Reagent, a blend of lipids in non-liposomal form and other compounds in 80 % ethanol, obtainable from Roche Diagnostics Corporation. Viral vectors comprising effector genes are generally described in the publications referenced in the last section.

The cells to be transfected have to be able to differentiate into at least one particular cell type. This will be the case for the precursor cell for the desired cell type. Precursor cells include, but are not limited to, for example, the human neuronal precursor cell line NTERA-2 cl.D1. (Leypoldt et al., J. Neurochem. 76 (2001), 806-814), the human retinal precursor cell line KGLDMSM (Ezeonu et al., DNA Cell Biol. 19 (2000), 527-537), a human leukemic cell line, PER-117, bearing the markers of a T-cell precursor phenotype (Kees, Blood 72 (1988), 1524-1529) or HL60, a promyelocytic leukemia cell line that differentiates to mature neutrophils (Fontana, Proc. Natl. Acad. Sci. USA 77 (1980), 3664-3668).

Since these precursor cell lines have only a limited range of developmental capacity it is preferred that multipotent or pluripotent cells are used. This is the case when said cells are or are derived from stem cells or primordial germ cells, which are preferred in the embodiments of the present invention.

Any part of the description relating to human embryonic stem cells which could be prepared exclusively by a method that necessarily involves the destruction of human embryos is not part of the invention. References thereto are solely for descriptive purposes.

The invention can be practiced using stem cells of any vertebrate species. Included are stem cells from humans as well as non-human primates, domestic animals, livestock, and other non-human mammals. Preferred sources for stem cells are rodents, in particular mice and rats. Amongst the stem cells suitable for use in this invention are primate pluripotent stem cells derived from tissue formed after gestation, such as a blastocyst, or fetal or embryonic tissue taken any time during gestation. Non-limiting examples are established lines of embryonic stem cells. The invention is also applicable to adult stem cells, for example multipotent adult progenitor cells (MAPCs); see, e.g., Reyes and Verfaillie, Ann. N Y Acad. Sci. 938 (2001), 231-235 and Weissman and Anderson, Annu. Rev. Cell Dev. Biol. 17 (2001), 387-403. It is also referred to the literature of Anderson et al., Nat. Med. 7 (2001), 393-395, and Prockop, Science 276 (1997), 71-74, wherein the extraction and culture of adult stem cells is described.

Media for isolating and propagating stem cells can have any of several different formulas, as long as the cells obtained have the desired characteristics, and can be propagated further. Suitable sources include Iscove's modified Dulbecco's medium (IMDM), Gibco, #12440-053; Dulbecco's modified Eagles medium (DMEM), Gibco #11965-092; Knockout Dulbecco's modified Eagles medium (KO DMEM), Gibco #10829-018; 200 mM L-glutamine, Gibco # 15039-027; non-essential amino acid solution, Gibco 11140-050; [beta]-mercaptoethanol, Sigma # M7522; human recombinant basic fibroblast growth factor (bFGF), Gibco # 13256-029. Exemplary serum-containing ES medium and conditions for culturing stem cells are known, and can be optimized appropriately according to the cell type. Media and culture techniques for particular cell types referred to in the previous section are provided in the references cited herein.

Because of their wide range of developmental capacity said stem cells are embryonic stem (ES) cells in a particularly preferred embodiment of this invention.

As mentioned before, several sources for ES cells are at the disposal of the skilled person of which human stem cells are preferred for most of the embodiments of the present invention. Human embryonic stem cells and their use for preparing different cell and tissue types are also described in Reprod. Biomed. Online 4 (2002), 58-63. Embryonic stem cells can be isolated from blastocysts of members of the primate species (Thomson et al., Proc. Natl. Acad. Sci. USA 92 (1995), 7844). Human embryonic germ (EG) cells can be prepared from primordial germ cells present in human fetal material taken about 8-11 weeks after the last menstrual period. Suitable preparation methods are described in Shamblott et al., Proc. Natl. Acad. Sci. USA 95 (1998), 13726. Methods for making cells that resemble embryonic stem cells or embryonic germ cells in morphology and pluripotency derived from primordial germ cells isolated from human embryonic tissue, such as from the gonadal ridges of human embryo, are described in US patent US-A-6,245,566.

Recently, is has been reported that exfoliated human deciduous tooth, a comparably very accessible tissue, contains multipotent stem cells that were identified to be a population of highly proliferative, clonogenic cells capable of differentiating into a variety of cell types including neural cells, adipocytes, and odontoblasts; see Miura et al., Proc. Natl. Acad. Sci. USA 100 (2003), 5807-5812. After in vivo transplantation, those cells were found to be able to induce bone formation, generate dentin, and survive in mouse brain along with the expression of neural markers. Furthermore, multilineage potential of homozygous stem cells derived from metaphase II oocytes has been described by Lin et al. in Stem Cells 21 (2003), 152-161. Various sources of precursor cells in postnatal muscles and the factors that may enhance stem cell participation in the formation of new skeletal and cardiac muscles in vivo are reviewed in Grounds et al. J. Histochem. Cytochem. 50 (2002), 589-610. Purification of rare hematopoietic stem cell(s) (HSC) to homogeneity that home to bone marrow is described in US patent application US2003/0032185. These adult bone marrow cells are described to have tremendous differentiative capacity as they can also differentiate into epithelial cells of the liver, lung, gastro-intestinal tract, and skin. This finding may contribute to clinical treatment of genetic diseases or tissue repair. Furthermore, techniques such as nuclear transfer for embryo reconstruction may be employed, wherein diploid donor nuclei are transplanted into enucleated MII oocytes. This technology along with other procedures that aid in the establishment of customized embryonic stem (ES) cell lines that are genetically identical to those of the recipient have been reviewed by Colman and Kind, Trends Biotechnol. 18 (2000), 192-196.

The field of stem cell technology is being reviewed by Kiessling and Anderson, Harvard Medical School, in Human Embryonic Stem Cells: An Introduction to the Science and Therapeutic Potential; (2003) Jones and Bartlett Publishers; ISBN: 076372341X.

In order to avoid the use of, for example, human embryos as the donor for stem cells, it may be possible to employ transgenic non-human animals, in particular mammals as source for embryonic stem cells. For example, compositions and methods for making transgenic swines to be used as xenograft donors are described in US patent US-A-5,523,226. Likewise, WO97/12035 describes methods of producing transgenic animals for xenotransplantation. Furthermore, immunologically compatible animal tissue, suitable for xenotransplantation into human patients, is described in WO01/88096. Methods for making embryonic germ cells from porcine are described for example in US patent US-A-6,545,199. Cells immunologically compatible with humans can also be employed for purposes of the present invention.

Stem cells can be propagated continuously in culture, using a combination of culture conditions that promote proliferation without promoting differentiation. Traditionally, stem cells are cultured on a layer of feeder cells, typically fibroblast type cells, often derived from embryonic or fetal tissue. The cell lines are plated to near confluence, usually irradiated to prevent proliferation, and then used to support when cultured in a medium conditioned by certain cells (e.g. Koopman and Cotton, Exp. Cell 154 (1984), 233-242; Smith and Hooper, Devel. Biol. 121 (1987), 1-91), or by the exogenous addition of leukemia inhibitory factor (LIF). Such cells can be grown relatively indefinitely using the appropriate culture conditions without differentiation.

In the absence of feeder cells, exogenous leukemia inhibitory factor (LIF), or conditioned medium, ES or EG cells spontaneously differentiate into a wide variety of cell types, including cells found in each of the endoderm, mesoderm, and ectoderm germ layers. With the appropriate combinations of growth and differentiation factors, however, cell differentiation can be controlled. For example, mouse ES and EG cells can generate cells of the hematopoietic lineage in vitro (Keller et al., Mol. Cell Biol. 13 (1993), 473-486; Palacios et al., Proc. Natl. Acad. Sci. USA 92 (1995), 7530-7534; Rich, Blood 86 (1995), 463-472). Additionally, mouse ES cells have been used to generate in vitro cultures of neurons (Bain et al., Developmental Biology 168 (1995), 342-357; Fraichard et al., J. Cell Science 108 (1995), 3161-3188), cardiomyocytes (heart muscle cells) (Klug et al., Am. J. Physiol. 269 (1995), H1913-H1921), skeletal muscle cells (Rohwedel et al., Dev. Biol. 164 (1994), 87-101), vascular cells (Wang et al., Development 114 (1992), 303-316). US patent US-A-5,773,255 relates to glucose-responsive insulin-secreting pancreatic beta cell lines, US patent US-A-5,789,246 relates to hepatocyte precursor cells. Hepatic differentiation of murine embryonic stem cells is also described in Jones et al., Exp. Cell Res. 272 (2002), 15-22.

Other progenitors of interest include, but are not limited to chondrocytes, osteoblasts, retinal pigment epithelial cells, fibroblasts, skin cells such as keratinocytes, dendritic cells, hair follicle cells, renal duct epithelial cells, smooth and skeletal muscle cells, testicular progenitors, and vascular endothelial cells.

In certain embodiments of the invention, differentiation is promoted by withdrawing one or more medium component(s) that promote(s) growth of undifferentiated cells, or act(s) as an inhibitor of differentiation. Examples of such components include certain growth factors, mitogens, leukocyte inhibitory factor (LIF), and basic fibroblast growth factor (bFGF). Differentiation may also be promoted by adding a medium component that promotes differentiation towards the desired cell lineage, or inhibits the growth of cells with undesired characteristics.

In a preferred embodiment of the present invention, said reporter gene in the recombinant nucleic acid molecule described above is operably linked to at least one cell type-specific regulatory sequence. This embodiment has the advantage of providing not only a monitoring system for differentiation per se but also allows observing the differentiation into a certain cell type. The regulatory sequence will drive the expression of the reporter molecule only in those cells that acquire the desired cell type by differentiation. Usually, said regulatory sequence comprises such promoter and/or enhancer elements. In addition, or alternatively, a second reporter gene can be used which is under the control of said cell type-specific regulatory sequence. The second reporter gene may be the same as used for the differentiation-dependent expression or different, including conventional reporter genes such as green fluorescent protein (GFP). Furthermore, the second reporter gene may be present in the mentioned recombinant nucleic acid molecule or on a different vector construct, the latter option requiring co-transfection of both molecules into the cells.

In a further embodiment, the differentiated cells containing the recombinant nucleic acid molecule of the present invention, and optionally further foreign DNA such as vector constructs described above, are enriched and separated from non-differentiated cells and/or from cells of non-desired cell types. Thus, in accordance with this invention, populations of differentiated cells are depleted of relatively undifferentiated cells and/or of cells of undesired cell types by using a selection system that is lethal to the undesired cells and cell types. This can be achieved, for example, by expressing a selectable marker gene under the control of a regulatory sequence that causes the gene to be preferentially expressed in the desired cell type and/or at a certain stage of development, which renders cells of a specific cell type resistant to a lethal effect of an external agent. To accomplish this, the cells are genetically altered with a recombinant nucleic acid molecule comprising the selectable marker gene before the process used to differentiate the cells into the desired lineage. The selection system may be present in the recombinant nucleic acid molecule of the present invention or on a different vector construct which is used for co-transfection.

The cell types which the differentiation can be directed to, include connecting fibroblasts, stromal cells, endothelial cells, glia cells, neural cells, neuronal cells, hematopoietic cells, smooth muscle cells, skeletal muscle cells, epithelial cells, and cardiac cells. To monitor differentiation into these particular cell types, regulatory sequences have to be operatively linked to the reporter gene sequence that confers an expression of the reporter which is specific for said cell types.

In a preferred embodiment of the method according to the invention, said promoter or enhancer is selected from the group consisting of αMHC, MLC2V, VE-cadherin, Tie-2, Flk-1, Flt-1, GFAP, alpha-1 tubulin and collagen 2 promoter or enhancer. Whereas the pro-alphal (II) collagen chain (collagen 2) promoter/enhancer (Zhou et al., J. Cell Sci. 108 (1995), 3677-3684) is specific for chondrocytes, the expression in endothelial cells can be driven by the VE-cad promoter (Gory et al., Blood 93 (1999), 184-192), the Flt-1 promoter (Quinn et al., Biochem. Biophys. Res. Commun. 276 (2000), 1089-1099), the Tie-2 (Schlaeger et al., Proc. Natl. Acad. Sci. USA 94 (1997), 3058-3063) and the Flk-1 promoter/enhancer (Kappel et al., Blood 93 (1999), 4284-4292), respectively. The alpha-1 tubulin promoter (Gloster et al., J. Neurosci. 14 (1994), 7319-7330) and the glial fibrillary acidic protein (GFAP) promoter (Besnard et al., J. Biol. Chem. 266 (1991), 18877-18883) are specific for neural cells. The smooth muscle heavy chain (SMHC) minimal promoter is specific for smooth muscles (Kallmeier et al., J. Biol. Chem. 270 (1995), 30949-30957). An HSV-1 vector containing the rat tyrosine hydroxylase promoter for use in both long-term and cell type-specific expression in the midbrain is described by Song et al., in J. Neurochem. 68 (1997), 792-803. Cardiac-specific promoters such as alpha-myosin heavy chain promoter (αMHC) and the myosin light chain-2v (MLC2v) promoter, the latter being specific for ventricular heart muscles, are described further below.

Further examples for tissue-specific promoters are those which are active in hematopoietic cells, cartilage cells or epidermal cells as well as insulin-secreting β-cells.

"Tissue-specific" is to be subsumed under the term "cell-specific". Regulatory sequences of the above described cell types and other cell type-specific promoters can be obtained from the literature; see, e.g., "medline" and NCBI.

Cardiovascular diseases are still among the most frequent causes of death in the western industrial countries. Only through intensive basic research in this field, the pathophysiological causes can be found, and new therapeutic approaches can be devised, and toxicological changes described. For studying the pathogenesis of cardiovascular diseases and for testing new pharmacological and toxicological substances, models are needed which, on one hand, can be transferred to humans and, on the other hand, can replace the tedious and cost-intensive animal models. Still in the year 1991, over 2 million animals have been employed for animal experiments in the federal states of the former Western Germany alone.

One starting point of pharmacological and toxicological research which has recently grown more and more important is heart differentiation. From the stereotypically proceeding heart cell development, conclusions can be drawn to pathological and toxicological changes of cardiomyocytes. Thus, for example, it is known that the receptor state and the intracellular signal cascades are disturbed in cardiac hypertrophy (Yamazaki et al., J. Mol. Cell Cardiol. 27 (1995), 133-140) and heart insufficiency (Johnson et al., Biochem. Pharmacol. 45 (1993), 2365-2372). These pathologically altered cardiomyocytes are in part again similar to heart cells of early differentiation stages. Thus, besides toxicological tests assays for the identification and isolation of substances that are capable of promoting cardiac remodeling are needed, which can be used for therapeutic interventions in the treatment of patients with cardiac dysfunction characterized by, for example, pathological hypertrophy and fibrosis.

However, the examinations necessary for elucidating the properties of heart cells in early differentiation stages are technically difficult to perform with live animals and, if they can be performed at all, it is only in very complicated studies: On day 12-13, at the earliest, it is possible to prepare cardiomyocytes from a murine embryo, but such cells no longer correspond to an early cardiac differentiation stage. A detailed analysis of receptor expression during various differentiation stages requires a very high expense of animal material and is technically difficult to perform as set forth above. Similarly, the observation of the development of a relatively undifferentiated heart cell over several days or weeks is not possible with an animal model. In order to test the use of novel therapeutical agents, e.g., inotropic substances or antiarrhythmics or toxical substances, e.g., heavy metals or retinoids, an invasive monitoring of animals, e.g., swine, must be performed for weeks in animal experiments.

Now, it has been the object of the present invention to provide a cell-based assay which enables a simple characterization of the effects of compounds on cardiomyogenesis and allows for functional examinations rather than being based on reporter genes such as Lac-Z (Niwa et al., Gene 108 (1991), 193-199; Wobus et al., J. Mol. Cell Cardiol. 29 (1997), 1525-1539; Metzger et al., Circ. Res. 78 (1996), 547-552), as with prior methods, the expression of which genes can be detected only after cell fixation and by means of a specific substrate.

Surprisingly, it has been found that ES cells can be stably transfected with a DNA construct in which a gene encoding a secreted reporter protein is coupled with a cardiomyocyte-specific cell- and development-dependent promoter; see the examples and the vector sequence depicted in SEQ ID NO: 3. This construct is integrated in the native DNA and after the specific activation of intracellular signals, the promoter is activated and the reporter protein expressed and secreted into the medium. Thus, ES cells which activate a cell-specific transcription factor at a certain point of differentiation could be recognized by determining the level of reporter protein or its activity in the cell culture medium.

The purpose of the method of the present invention as illustrated in the examples carries with it that the reporter molecule employed should preferably truly be secreted into the cell culture medium, or in case of a transgenic non-human animal, into the body fluid. Otherwise, if the once secreted reporter molecule would be recaptured from the medium and the body fluid, respectively, it would hardly be possible to quantify the differentiated cells, since the measured amount of activity or active protein in the cell culture supernatant or body fluid might not properly or at all correspond to the actual number of differentiated cells and/or the status of differentiation. Therefore, reporter molecules such as human alpha-galactosidase which, when correctly glycosylated, can be taken up by the cells via mannose-6 phosphate receptors, would probaby at least in its native form not be a suitable reporter molecule for the purposes of the present invention. Nevertheless, this enzyme may be rendered suitable for the purposes of the present invention, e.g., by abolishing its capability of being glycosylated, for example by means of corresponding mutations in the underlying DNA encoding the enzyme.

In a particularly preferred embodiment of the invention, said differentiating cell type are cardiomyocytes. For this embodiment, said cell type-specific regulatory sequence is preferably atrial- and/or ventricular-specific. Corresponding regulatory sequences, i.e. cardiac-specific promoters are described in the prior art; see also supra. For example, Nkx-2.5 specific for very early cardiomyocytes and mesodermal precursor cells, respectively (Lints et al., Development 119 (1993), 419 431); human-cardiac-α-actin specific for heart tissue, (Sartorelli et al., Genes Dev. 4 (1990), 1811-1822), and MLC-2V specific for ventricular heart muscle cells (O'Brien et al., Proc. Natl. Acad. Sci. USA. 90 (1993), 5157 5161; Lee et al., Mol. Cell Biol. 14 (1994), 1220-1229; Franz et al., Circ. Res. 73 (1993), 629-638 and WO96/16163). The cardiac-specific alpha-myosin heavy chain promoter is described in Palermo et al., Cell Mol. Biol. Res. 41 (1995), 501-519 and Gulick et al., J. Biol. Chem. 266 (1991), 9180-91855. The expression of the atrial-specific myosin heavy chain AMHCl and the establishment of anteroposterior polarity in the developing chicken heart is described in Yutzey et al., Development 120 (1994), 871-883.

Since the detection is aided by using a reporter molecule for which a robust, sensitive and specific assay exists, a particularly preferred embodiment is the method according to the invention, wherein said reporter gene product is secreted alkaline phosphatase (SEAP), alpha-amylase or invertase.

SEAP is heat-resistant and not inhibited by L-homoarginine. Further advantages of this reporter molecule are its low toxicity and the fact that the protein levels that are secreted correspond directly to the amount of intracellular mRNA. Thus, the SEAP reporter assay provides a direct measurement for the cell type-specific expression and thereby the differentiation. It is also extremely sensitive: it is possible to detect 10⁻¹³g SEAP protein (Yang et al., Biotechniques 23 (1997), 1110-1114). This makes it possible to detect differentiation into cell types that are rare.

The use of alpha-amylase as reporter molecule is described in, for example, WO98/49320. This reporter molecule is particularly attractive because the enzyme is extremely stable over a wide range of conditions. The measurement of alpha-amylase activity is simple, quantitative, sensitive, safe and inexpensive. It was also shown to be compatible with a SEAP reporter system (Schlatter et al., Gene 282 (2002), 19-31). The use of invertase as a marker protein is described for example in Mace et al., Virology 188 (1992), 869-874.

Reporter gene constructs comprising inter alia secreted alkaline phosphatase as the reporter molecule and which therefore can be used as a source for preparing vector constructs according to the present invention are described for example in WO00/34435 and Wang et al., Gene 279 (2001), 99-108, the latter describing murine SEAP. Detection of SEAP level can be performed as described in the examples and in the prior art; see, e.g., Bronstein et al., Biotechniques 17 (1994), 172-177; Yang et al. (1997) and Wang et al. (2001), supra. The use of human secreted alkaline phosphatase in an in vivo mouse reporter gene model to monitor ovarian tumor growth and response to therapeutics has been described in Nilsson et al., Cancer Chemother. Pharmacol. 49 (2002), 93-100. This model can be adapted to the embodiments of the present invention. Engineered secreted alkaline phosphatase reporter genes being capable of being expressed in a mammal for extended periods of time and detection of the alkaline phosphatase activity by measuring the protein levels or AP activity of a serum or tissue sample from a mammal containing the cell with the transgene, or measuring the media from a cultured cell containing the transgene are also described in WO02/095068. Reporter activity or protein amount, in particular SEAP activity, can be detected in any biological fluid such as breast milk, amniotic fluid, seminal plasma, serum and urine.

Since not all molecules that can be used as a reporter are secreted naturally, the nucleic acid molecule introduced into the cells can be genetically engineered to encode a protein that is excreted. Particularly preferred is the method described above, wherein said reporter gene product comprises a secretory leader sequence. Examples for secretory leader sequences are but are not limited to the adenovirus tripartite leader sequence (Chu et al., Biotechniques 5 (1995), 890-896), the immunoglobulin secretion signal (Schlatter et al., Gene 282 (2002), 19-31), the SUC2 signal sequence as well as the human interleukin 2 receptor alpha chain signal sequence (Kamiya et al., Tohoku J. Exp. Med. 180 (1996), 297-308).

In order to select those cells which contain the recombinant nucleic acid molecule according to the invention, and optionally other vector constructs such as those described hereinbefore, said recombinant nucleic acid molecules and vector constructs, respectively, preferably further comprise a selectable marker expressed by multi- or pluripotent cells. Selectable markers are known to the person skilled in the art and include, for example, nucleoside and aminoglycoside-antibiotic-resistance genes conferring resistance to, e.g., puromycin, neomycin or hygromycin. Further examples for resistance genes are dehydrofolate-reductase, which confers a resistance against aminopterine and methotrexate, as well as multi drug resistance genes, which confer a resistance against a number of antibiotics, e.g. against vinblastin, doxorubicin and actinomycin D; see, e.g., Blau et al., Blood 89 (1997), 146-154, which describes the use of the multiple drug resistance gene 1 (MDR1) gene as a dominant selectable marker.

In a particularly preferred embodiment of the present invention, said selectable marker confers resistance to puromycin. Puromycin is particularly suited for the fast elimination of non-cardiac cells in adherent culture of transgenic EBs. Furthermore, drug selection of cardiac cells can be implemented entirely in the suspension culture of transgenic EBs. Hence, it could also be shown that purified ES cell-derived cardiomyocytes survive much longer in culture than untreated counterparts. Moreover, the elimination of undifferentiated ES cells during drug selection process has itself been shown to have a clear positive effect on viability and longevity of such differentiated ES cell-derived cells as cardiomyocytes. In addition, it could be surprisingly shown that the release from surrounding non-differentiated cells induces proliferation of cardiomyocytes. Thus, the drug selection possesses both a purifying and multiplying effect; see, e.g., WO02/051987. Thus, the selection does not only provide the means for eliminating cells which can not be monitored since they do not carry the reporter gene, it also allows the elimination of cells that did not differentiate into the desired cell type, provided that the reporter gene and the selectable marker gene are driven by the same, cell type-specific regulatory sequence.

The method according to the invention, wherein said cells form cell aggregates or tissue-like aggregates derived from different ES cell types is a preferred embodiment, and particularly preferred are said cells forming embryoid bodies (EBs). As mentioned before, embryoid bodies represent a complex group of cells differentiating into different tissue. In one embodiment, the cells within an embryoid body are substantially synchronized for their differentiation. Accordingly, at known intervals, the majority of the synchronized cells differentiate into the three embryonic germ layers and further differentiate into multiple tissue types, such as cartilage, bone, smooth and striated muscle, and neural tissue, including embryonic ganglia; see also Snodgrass et al., "Embryonic Stem Cells: Research and Clinical Potentials" in Smith and Sacher, eds. Peripheral Blood Stem Cells American Association of Blood Banks, Bethesda MD (1993). Thus, the cells within embryoid bodies provide a much closer model to the complexity of whole organisms than do traditional single cell or yeast assays, while still avoiding the cost and difficulties associated with the use of mice or rats and larger mammals. Moreover, the recent availability of human embryoid bodies improves the predictive abilities of the invention by providing an even closer vehicle for modeling toxicity and identification of drugs useful for the treatment of heart disorders in human organ systems, and in humans.

The embryoid body of the invention comprises a cell population, the majority of which being pluripotent cells capable of developing into different cellular lineages when cultured under appropriate conditions. It is preferred that the embryoid body comprises at least 51 % pluripotent cells derived from totipotent ES cells. More preferably, the embryoid body comprises at least 75 % pluripotent cells derived from totipotent ES cells. And still more preferably, the embryoid body comprises at least 95 % pluripotent cells derived from totipotent ES cells.

In its simplest form, the method of creating a molecular profile according to the present invention involves contacting embryoid bodies with a chemical composition of interest, and then determining the alterations in reporter activity or amount of reporter protein, or both, in the embryoid body exposed to the chemical composition (the "test embryoid body") compared to a embryoid body which was not exposed to the agent (a "control embryoid body").

Thus, the method of the present invention can be performed such that it allows self-assembly of the cells into the aggregates or tissue-like structures. The term "embryoid bodies" (EBs) is a term of art synonymous with "aggregate bodies". The terms refer to aggregates of differentiated and undifferentiated cells that appear when ES cells overgrow in monolayer cultures, or are maintained in suspension cultures. Embryoid bodies are a mixture of different cell types, typically from several germ layers, distinguishable by morphological criteria. WO02/051987 describes a protocol to obtain embryoid bodies. The manufacturing takes place preferably with the "hanging drop" method or by methylcellulose culture (Wobus et al., Differentiation 48 (1991), 172-182).

Alternatively to this, spinner flasks (stirring cultures) can be used as culture method. Therefore, the undifferentiated ES cells are introduced into stirring cultures and are mixed permanently according to an established procedure. Therefore, 10 million ES cells are introduced into 150 ml medium with 20 % FCS and are stirred constantly with the rate of 20 rpm, wherein the direction of the stirring motion is changed regularly. 24 hours after introduction of the ES cells an extra 100 ml medium with serum is added and thereupon 100-150 ml of the medium is exchanged every day (Wartenberg et al., FASEB J. 15 (2001), 995-1005). Under these culture conditions large amounts of ES cell-derived cells, i.e. cardiomyocytes, endothelial cells, neurons etc., depending on the composition of the medium, can be obtained. The cells are selected by means of the resistance gene either still within the stirring culture or after plating, respectively.

In a particularly preferred embodiment of the present invention, embryoid bodies are prepared according to a recently developed "mass culture" system employed in the appended examples and described in detail in international application WO2005/005621.

The embryoid bodies used to test the chemical composition can be of any vertebrate species. The choice of the particular species from which the embryoid body is derived will typically reflect a balance of several factors. First, depending on the purpose of the study, one or more species may be of particular interest. For example, human embryoid bodies will be of particular interest for use with compositions being tested as potential human therapeutics but also for toxicological tests for substances including industrial chemicals, while equine, feline, bovine, porcine, caprine, canine, or sheep embryoid bodies may be of more interest for a potential veterinary therapeutic. Embryoid bodies of other species commonly used in preclinical testing, such as guinea pigs, mice, rat, rabbits, pigs, and dogs, are also preferred. Typically, embryoid bodies of these species will be used for "first pass" screening, or where detailed information on toxicity in humans is not needed, or where a result in a murine or other one of these laboratory species has been correlated to a known toxicity or other effect in humans. Furthermore, with respect to human therapeutics, regulatory agencies generally require animal data before human trials can begin; it will generally be desirable to use embryoid bodies of species which will be used in the preclinical animal studies. The results of toxicity testing in the embryoid bodies can then guide the researcher on the degree and type of toxicity to anticipate during the animal trials. Certain animal species are known in the art to be better models of human toxicity of different types than are others, and species also differ in their ability to metabolize drugs; see, e.g., Williams, Environ. Health Perspect. 22 (1978), 133-138; Duncan, Adv. Sci. 23 (1967), 537-541. Thus, the particular species preferred for use in a particular preclinical toxicity study may vary according to the intended use of the drug candidate. For example, a species which provides a suitable model for a drug intended to affect the reproductive system may not be a suitable model for a drug intended to affect the nervous system. Criteria for selecting appropriate species for preclinical testing are well known in the art.

Once an embryoid body culture has been initiated, it can be contacted with a chemical composition. Conveniently, the chemical composition is in an aqueous solution, preferably in a solvent conventionally used in cell culture, for example DMSO, and is introduced to the culture medium; see also the examples. The introduction can be by any convenient means, but will usually be by means of a pipette, a micropipettor or a syringe. In some applications, such as high throughput screening, the chemical compositions will be introduced by automated means, such as automated pipetting systems, which may be on robotic arms. Chemical compositions can also be introduced into the medium as in powder or solid forms, with or without pharmaceutical excipients, binders, and other materials commonly used in pharmaceutical compositions, or with other carriers which might be employed in the intended use. For example, chemical compositions intended for use as agricultural chemicals or as petrochemical agents can be introduced into the medium by themselves to test the toxicity of those chemicals or agents, or introduced in combination with other materials with which they might be used or which might be found in the environment, to determine if the combination of the chemicals or agents has a synergistic effect. Typically, the cultures will be shaken at least briefly after introduction of a chemical composition to ensure that the composition is dispersed throughout the medium.

The time at which a chemical composition is added to the culture is within the discretion of the practitioner and will vary with the particular study objective. Conveniently, the chemical composition will be added as soon as the embryoid body develops from the stem cells, permitting the determination of the alteration in protein or gene expression on the development of all the tissues of the embryoid body. It may be of interest, however, to focus the study on the effect of the composition on a particular tissue type. As previously noted, individual tissue, such as muscle, nervous, and hepatic tissue, are known to develop at specific times after the embryoid body has formed. Addition of the chemical composition can therefore be staged to occur at the time the tissue of interest commences developing, or at a chosen time after commencement of that development, in order to observe the effect on altering gene or protein expression in the tissue of interest.

Different amounts of a chemical composition will be used to contact an embryoid body depending on the amount of information known about the toxicity of that composition, the purposes of the study, the time available, and the resources of the practitioner. A chemical composition can be administered at just one concentration, particularly where other studies or past work or field experience with the compound have indicated that a particular concentration is the one which is most commonly found in the body. More commonly, the chemical composition will be added in different concentrations to cultures of embryoid bodies run in parallel, so that the effects of the concentration differences on gene or protein expression and, hence, the differences in toxicity of the composition at different concentrations, can be assessed. Typically, for example, the chemical composition will be added at a normal or medium concentration, and bracketed by twofold or fivefold increases and decreases in concentration, depending on the degree of precision desired.

Where the composition is one of unknown toxicity, a preliminary study is conveniently first performed to determine the concentration ranges at which the composition will be tested. A variety of procedures for determining concentration dosages are known in the art. One common procedure, for example, is to determine the dosage at which the agent is directly toxic. The practitioner then reduces the dose by one half and performs a dosing study, typically by administering the agent of interest at fivefold or twofold dilutions of concentration to parallel cultures of cells of the type of interest. For environmental contaminants, the composition will usually also be tested at the concentration at which it is found in the environment. For agricultural chemicals, such as pesticides which leave residues on foodstuffs, the agent will usually be tested at the concentration at which the residue is found, although it will likely be tested at other concentrations as well. Thus, the dilution of test compounds can be done by making in separated tubes a series of dilution of 50 or 100 fold concentrated compounds in DMSO. One or two µl of each dilution are distributed in each well before cell suspension distribution. Particularly preferred are stock and dilution series as described for retinoic acid in Example 3.

The above considerations with respect to contacting the compounds with the EBs, contacting time, etc., also apply to the assays of the invention performed on, e.g., ES cells, tissue and non-human animals, if applicable.

The present invention also relates to reporter gene constructs for monitoring cell differentiation comprising the recombinant nucleic acid molecule defined previously as well as to cells capable of differentiating into at least one particular cell type, that contain said reporter gene construct, or are obtainable by the methods described herein. Likewise, cell aggregates, tissue, organs, implants or transplants comprising said reporter gene construct, obtainable by the methods described herein or constituted from said cells, cell aggregates, tissue, organs, implants or transplants are subject of the invention.

In a still further embodiment, the present invention relates to a transgenic non-human animal which can be generated from the mentioned ES cells and ES cell-derived cell types and cell aggregates; see supra. A method for the production of a transgenic non-human animal, which is encompassed by the present invention, for example transgenic mouse, comprises introduction of a reporter gene as described above into a germ cell, an embryonic cell, stem cell or an egg or a cell derived therefrom. The non-human animal can be used in accordance with a screening method of the invention described herein. Production of transgenic embryos and screening of those can be performed, e.g., as described by A. L. Joyner Ed., Gene Targeting, A Practical Approach (1993), Oxford University Press. A general method for making transgenic non-human animals is described in the art, see for example WO94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62 (1990), 1073-1085) essentially as described (Robertson, in Teratocarcinomas and Embryonic Stem Cells: A Practical Approach. E. J. Robertson, ed. (Oxford: IRL Press) (1987), 71-112) may be used for homologous gene targeting. Other suitable ES cell lines include, but are not limited to the E14 line (Hooper et al., Nature 326 (1987), 292-295), the D3 line (Doetschman et al., J. Embryol. Exp. Morph. 87 (1985), 27-45), the CCE line (Robertson et al., Nature 323 (1986), 445-448), the AK-7 line (Zhuang et al., Cell 77 (1994), 875-884) and others; see also supra. The success of generating a mouse line from ES cells bearing a specific targeted mutation depends on the pluripotence of the ES cells (i.e. their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born as chimeric mice. The resultant transgenic mice are backcrossed and screened for the presence of the correctly targeted transgene(s) by PCR or Southern blot analysis on tail biopsy DNA of offspring so as to identify transgenic mice for the reporter locus/loci. For example, transient expression of a recombinase gene directly introduced into fertilized eggs by pronuclear injection provides a fast and efficient method for generating mutant mice with desired deletions or translocations in target genes. Efficient removal of loxP-flanked DNA sequences in a gene-targeted locus by transient expression of Cre recombinase in fertilized eggs is described by Sunaga et al., Mol. Reprod. Dev. 46 (1997), 109-113.

Methods for producing transgenic flies, such as Drosophila melanogaster are also described in the art, see for example US-A-4,670,388, Brand and Perrimon, Development 118 (1993), 401-415; and Phelps and Brand, Methods 14 (1998), 367-379. Transgenic worms such as C. elegans can be generated as described in Mello et al., EMBO J. 10 (1991), 3959-3970, Plasterk, Methods Cell. Biol. 48 (1995), 59-80.

All of the embodiments described above can not only be used to monitor cell differentiation and optimize culturing conditions using modulators in their pure forms but the effective agent(s) might also be contained in a composition. Hence, compositions of matter comprising any one of those recombinant nucleic acid molecules, cells, cell aggregates, or tissue of the present invention as described herein are encompassed in the scope of the present invention. As described herein, those compositions and methods of the invention can be used for a variety of purposes, for example for analyzing early steps of tissue formation during embryonic development or the influence of factors and compounds on this process.

In a particularly preferred embodiment, the present invention relates to arrays and chips comprising a solid support and attached thereto or suspended thereon cells, cell aggregates or tissue obtained by the method of the present invention or being in the differentiation process. Such arrays generally consist of a substrate of glass, plastic or silicon on which the test cells aggregates or tissue are deposited in a particular pattern. Depending on the type of array, these might additionally be covered by a conductor, e.g., gold, platinum, indium-tin-oxide, iridium, etc., which allows a direct measurement by employing the conductivity of cells. The use of such planar microelectrode arrays for cultured cells and cell aggregates as biosensors is of particular interest.

Furthermore, the invention relates to an apparatus for analyzing said array, which will be able to measure the particular read-out of the assay employed, e.g., SEAP reaction product, in a quantitative manner and assign the results to the position on the array.

The system for monitoring cell differentiation according to the present invention can also be employed to identify and characterize substances that modify the differentiation process. Populations of differentiated cells and tissue suitable for human administration have to be identified after the differentiation process to avoid administering undifferentiated cells with high tumorigenic potential or cells differentiated into cells that are not needed for the particular therapeutic purpose. The positive identification of cells differentiated into a certain cell type allows also the optimization of culturing conditions that is necessary to obtain the relatively high numbers of differentiated cells needed for therapeutic purposes. Thus, there is a need to identify conditions for culturing ES cells that drive their differentiation into the desired direction.

Accordingly, a preferred embodiment of the invention is a method of obtaining and/or profiling a modulator of cell differentiation comprising:
- contacting a test sample comprising a cell, cell aggregate, tissue or organ or a non-human animal as defined above with a test substance; and
- determining the effect of the test substance on the amount of the reporter gene product or activity compared to a control sample or animal.

In particular, the methods of the invention can be used for toxicological, embryotoxic, mutagenic, and/or teratogenic in vitro tests; see supra. Another important aspect of the present invention is therefore a method of determining toxicity, preferably teratogenicity, embryotoxicity, chronic or acute toxicity of a compound comprising the steps of the methods described herein, wherein a reduced or enhanced level or activity of said reporter gene product is indicative for the toxicity of the compound.

The assays may be simple "yes/no" assays to determine whether there is a change in expression of the secreted reporter protein by detecting reporter, e.g., enzyme activity or the amount of reporter protein. The assay may be made quantitative by comparing the amount of activity and/or amount of reporter in a test sample with the levels of activity and/or amount in a standard sample. As described in the examples, the test compound or a plurality of test compounds are subjected to the test cell, preferably an embryoid body in different concentrations or dilution series, preferably at doses that correspond to physiological levels of the corresponding type of test compounds; see also Example 3. It is thus also possible to easily generate compound profiles in purpose similar to those described in WO00/34525. For example, two or more assays may be used, wherein for each assay the test cells or, e.g., embryoid bodies comprise recombinant DNA molecules with different cell- and/or development-specific promoters operably linked to the same or different reporters, preferably wherein each reporter is a secreted protein, e.g., enzyme, most preferably wherein one or each reporter is SEAP. Those assays can be performed in parallel or subsequently; or the results of one assay may be compared with the results of a corresponding assay performed elsewhere. Likewise, it is envisaged to provide and use cells which comprise two of the mentioned recombinant molecules but with different promoters and reporters as mentioned above. Thus, a molecular profile of a test chemical composition can be established by detecting the alterations in reporter gene activity or amount of reporter protein in, for example, embryoid bodies contacted by the test chemical composition as described in previous sections. Once the molecular profile of the test composition is determined, it can be compared to that of a chemical composition with predetermined toxicities or, preferably, to a library of molecular profiles of chemical compositions with predetermined toxicities. The outcome of such comparison provides information for one to predict the likelihood of whether the test composition is toxic, what type of toxicity, and how toxic it would be as compared to the other known toxic compositions.

For the purpose of practicing the invention, the predictions of toxicity of the test composition based on its molecular profiles in ES cells, tissue, etc., preferably EB cells does not have to be 100 % accurate. To have a major positive impact on the efficiency and costs of drug development, one only has to modestly increase the probability that the less toxic and thus more successful drug candidates are, for example, on the top half of a prioritized list of new drug leads.

Furthermore, in Example 3 it has been demonstrated that the concentration-dependent effect of the test compound on the reporter molecule activity in culture supernatants is consistent with the concentration-dependent inhibition of the generation of the differentiated cell type by the test compound. Therefore, it is also possible to compare a compound profile generated with a method of the present invention with a compound profile provided by different means, for example, if both profiles quantify the in vitro differentiated cells.

Since the cells and tissue obtained in accordance with the present invention more closely resemble the in vivo situation compared to conventional cell-based assays, the results obtained by the assays of the present invention are expected to correlate to in vivo teratogenicity or embryotoxicity of the tested compounds as well. The simple read-out of the reporter molecule assay is easier measured and interpreted than determining the influence of a substance by monitoring the embryonic and post-natal development of a test animal in all aspects and in different stages of development. This requires a number of animals to be sacrificed at different ages, whereas by employing the reporter molecule secreted in a developmental-specific manner samples of body fluids from the animal can be obtained repeatedly and allowing the monitoring over an extended time period up to the life-time of the animal. This also reduces the variability of test results.

Several test substances can be combined and either added simultaneously or sequentially to gain information about possible enhancing or quenching effects. Thus, a further aspect of the invention relates to the method described previously, wherein said contacting step further includes contacting said test sample or animal with at least one second test substance in the presence of said first test substance. Two or more substances tested in combination will provide information about their interaction in general.

A preferred embodiment of the methods according to the present invention involves adding a compound known to activate or inhibit the differentiation process in the culture medium or animal, particularly if this test substance is a therapeutic agent or a mixture thereof. This screening may be done, for example, either because the compound is known to have an effect on the differentiation of certain cell types and is tested to determine its potential for guiding the differentiation of other cell types, or because a compound designed to have effects elsewhere may have unintended side effects. The last aspect applies particularly to therapeutic agents.

Both aspects of drug screening can be adapted to compound library screening methods as well as screening industrial chemicals from the shelf. Thus, another embodiment of the invention encompasses the method described above, wherein preferably in a first screen said test substance is comprised in and subjected as a collection of test substances. Such a collection of test substances, usually a combinatorial compound library, can have a diversity of about 10³ to about 10⁵ and is successively reduced in running the method several times until the diversity has been reduced down to a desired factor, preferably down to one compound or particular class of compounds. Optionally the method of the present invention can be combined with other screening methods known in the art. Methods for the generation and use of peptidomimetic combinatorial libraries are described in the prior art, for example in Ostresh, Methods in Enzymology 267 (1996), 220-234 and Domer, Bioorg. Med. Chem. 4 (1996), 709-715. Drug discovery by dynamic combinatorial libraries is described, for example, in Nat. Rev. Drug Discov. 1 (2002), 26-36 and Drug Discov. Today 7 (2002), 117-125. Compounds identified using the method of the invention can be further evaluated in single experiments employing other methods of the present invention.

Compounds and candidate agents are obtained from a wide variety of sources including libraries of synthetic or natural compounds, or as mentioned industrial chemicals; see supra. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. For example, inhibition of tumor-induced angiogenesis and matrix-metalloproteinase expression in confrontation cultures of embryoid bodies and tumor spheroids by plant ingredients used in traditional Chinese medicine has been described by Wartenberg et al. in Lab. Invest. 83 (2003), 87-98.

A preferred embodiment of the screening method is performed on an array as provided by the invention.

The assay methods of the present invention can be in a conventional laboratory format or adapted for high throughput screening. The term "high throughput screening" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired. Examples of assay formats include 96-well, 384-well or more-well plates, levitating droplets, and "lab on a chip" microchannel chips used for liquid handling experiments. It is well known by those in the art that as miniaturization of plastic molds and liquid handling devices are advanced, or as improved assay devices are designed, that greater numbers of samples may be performed using the design of the present invention. In the method of the invention, said one or more cells or tissue are preferably contained in a container, for example in a well of a microtiter plate, which may be a 24-, 96-, 384- or 1586-well plate. Alternatively, the cells can be introduced into a microfluidics device, such as those provided by Caliper (Newton, MA, USA). In another preferred embodiment, the method of the present invention comprises taking 3, 4, 5, 7, 10 or more measurements, optionally at different positions within the container.

The methods according to the invention can also be performed using cells that are genetically engineered to (over)express or inhibit the expression of a target gene. Examples for genetically altered stem cells are described in WO03/014326, which provides mammalian stem cells expressing the VEGF receptor VEGFR-1, or WO95/06409 disclosing human hematopoietic stem cells expressing recombinant DNA constructs encoding a recombinant molecule containing a signal transducing region and an antigenic specificity region. Alternatively to using already altered stem cells, said target gene can be transfected at the same time as the construct of the present invention conferring the expression of the secreted reporter molecule.

HTS, combinatorial library screening as well as conventional assay formats can also be employed to identify novel compounds which have an effect on differentiation. Novel as well as compounds previously known to modulate cell differentiation can be used to manufacture a drug for this purpose. Thus, a preferred embodiment of the present invention is a method of obtaining and manufacturing a drug which promotes or inhibits formation of specific cell types comprising the steps of the methods described above, wherein an enhanced or reduced level or activity of the reporter gene product is indicative for the drug.

Compounds of interest encompass numerous chemical classes, though typically they are organic molecules. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

The compounds may also be included in a sample including fluids to which additional components have been added, for example components that affect the ionic strength, pH, total protein concentration, etc. In addition, the samples may be treated to achieve at least partial fractionation or concentration. Biological samples may be stored if care is taken to reduce degradation of the compound, e.g., under nitrogen, frozen, or a combination thereof. The volume of sample used is sufficient to allow for measurable detection, usually from about 0.1 µl to 1 ml of a biological sample is sufficient.

Differentiation processes do not only play a major role in embryonic development but are also important for an adult organism since the continuous replacement of senescent or cells damaged by injury or disease is achieved by precursor cells differentiating into the cells needed. An important aspect of the present invention is thus a method of manufacturing an agent which supports wound healing and/or healing of damaged tissue comprising the steps described herein, wherein an enhanced level or activity of the reporter gene product is indicative for said agent.

Compounds identified, isolated and/or produced by the methods described above can also be used as lead compounds in drug discovery and preparation of drugs or prodrugs. This usually involves modifying the lead compound or a derivative thereof or an isolated compound as described hereinbefore such as modifying said substance to alter, eliminate and/or derivatize a portion thereof suspected causing toxicity, increasing bioavailability, solubility and/or half-life. Accordingly, a preferred embodiment of the present invention encompasses the methods described previously and further modifying said substance to alter, eliminate and/or derivatize a portion thereof suspected causing toxicity, increasing bioavailability, solubility and/or half-life. Methods for clinical compound discovery comprise for example ultrahigh-throughput screening (Sundberg, Curr. Opin. Biotechnol. 11 (2000), 47-53) for lead identification, and structure-based drug design (Verlinde and Hol, Structure 2 (1994), 577-587) and combinatorial chemistry (Salemme et al., Structure 15 (1997), 319-324) for lead optimization. The various steps recited above are generally known in the art. For example, computer programs for implementing these techniques are available, e.g., Rein, Computer-Assisted Modeling of Receptor-Ligand Interactions (Alan Liss, New York, 1989). Methods for the preparation of chemical derivatives and analogs are well known to those skilled in the art and are described in, for example, Beilstein, Handbook of Organic Chemistry, Springer edition New York Inc., 175 Fifth Avenue, New York, N.Y. 10010 U.S.A., and Organic Synthesis, Wiley, New York, USA. Furthermore, peptidomimetics and/or computer-aided design of appropriate derivatives and analogs can be used, for example, according to the methods described above. Methods for the lead generation in drug discovery also include using proteins and detection methods such as mass spectrometry (Cheng et al. J. Am. Chem. Soc. 117 (1995), 8859-8860) and some nuclear magnetic resonance (NMR) methods (Fejzo et al., Chem. Biol. 6 (1999), 755-769; Lin et al., J. Org. Chem. 62 (1997), 8930-8931). They may also include or rely on quantitative structure-action relationship (QSAR) analyses (Kubinyi, J. Med. Chem. 41 (1993), 2553-2564, Kubinyi, Pharm. Unserer Zeit 23 (1994), 281-290) combinatorial biochemistry, classical chemistry and others; see, for example, Holzgrabe and Bechtold, Pharm. Acta Helv. 74 (2000), 149-155.

In one embodiment, the method of the invention further comprises mixing the substance isolated or modified with a pharmaceutically acceptable carrier. Examples of carriers and methods of formulation may be found in Remington's Pharmaceutical Sciences.

Substances are metabolized after their in vivo administration in order to be eliminated either by excretion or by metabolism to one or more active or inactive metabolites (Meyer, J. Pharmacokinet. Biopharm. 24 (1996), 449-459). Thus, rather than using the actual compound or drug identified and obtained in accordance with the methods of the present invention, a corresponding formulation as a pro-drug can be used which is converted into its active form in the patient by his/her metabolism. Precautionary measures that may be taken for the application of pro-drugs and drugs are described in the literature; see, for review, Ozama, J. Toxicol. Sci. 21 (1996), 323-329.

The present invention also relates to a kit containing specific reagents such as those described hereinbefore useful for conducting any one of the above described methods of the present invention, containing, for example, the reporter gene construct described hereinbefore, multi-or pluripotent cells, standard compounds, and optionally culture medium, recombinant nucleic such acid molecules, detection means for the reporter molecule, in particular for SEAP such as EDTA, L-Homoarginine, NBT/BCIP, fixative solution, incubation buffer, PBS, etc. Such a kit would typically comprise a compartmentalized carrier suitable to hold in close confinement at least one container. The carrier would further comprise reagents useful for performing said methods. The carrier may also contain a means for detection such as labeled enzyme substrates or the like.

Yet another aspect of the present invention relates to a method of conducting a drug discovery business, comprising:
- providing one or more assay systems as described herein for identifying a modulator of cell differentiation; and/or
- conducting therapeutic profiling of modulators identified in the previous step, or further analogs thereof, for efficacy and toxicity in animals according to the invention; and
- formulating a pharmaceutical preparation including one or more modulators identified in the previous step as having an acceptable therapeutic profile.

Utilizing the methods described above, the identity of a modulator of differentiation or a derivative thereof are determined. Agents are identified by their ability to alter the level of secreted reporter molecule. For suitable lead compounds that are identified, further therapeutic profiling of the agent, or analogs thereof, can be carried out for assessing efficacy and toxicity in animals. Those compounds having therapeutic profiles after animal testing can be formulated into pharmaceutical preparations for use in humans or for veterinary uses. The subject business method can include an additional step of establishing a distribution system for distributing the pharmaceutical preparation for sale, and may optionally include establishing a sales group for marketing the pharmaceutical preparation.

Instead of developing the identified cell differentiation modulating agents in house, further drug development can also be achieved by a different company. Thus, a further aspect of the present invention relates to a method of conducting a target discovery business comprising:
- providing one or more assay systems described herein for identifying modulators of cell differentiation;
- (optionally) conducting therapeutic profiling of modulators identified in the previous step for efficacy and toxicity in animals according to the invention; and
- licensing, to a third party, the rights for further drug development and/or sales for modulators identified in the first step, or analogs thereof.

For suitable lead compounds that have been identified, further profiling of the agent, or further analogs thereof, can be carried out for assessing efficacy and toxicity in animals, depending on the modalities of the agreement with the respective third party. Further development of those compounds for use in humans or for veterinary uses will then be conducted by the third party. The subject business method will usually involve either the sale or licensing of the rights to develop said compound but may also be conducted as a service, offered to drug developing companies for a fee.

The present invention also relates to modulators of cell differentiation identified according to the methods described above as well as to pharmaceutical compositions for use in the modulation of cell differentiation comprising such a modulator.

The modulator according to the invention can be combined with suitable diluents or carriers, preferably those which are pharmaceutically acceptable. Examples of such carriers, diluents and methods of formulation may be found in Remington's Pharmaceutical Sciences. To form a pharmaceutically acceptable composition suitable for effective administration, such compositions will contain an effective amount of the modulator. Carriers or diluents are usually sterile and non-toxic, and defined as vehicles commonly used to formulate pharmaceutical compositions for animal or human administration. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like. A therapeutically effective dose refers to that amount of modulator which is sufficient to achieve the desired effect on differentiation of target cells.

Further examples of suitable pharmaceutical carriers are well known in the art and include phosphate-buffered saline solutions, emulsions, such as oil/water emulsions, various types of wetting agents, sterile solutions etc. Compositions comprising such carriers can be formulated by well known conventional methods. Accordingly, the present invention also provides a method of making a pharmaceutical composition for use in modulating cell differentiation comprising mixing a modulator of cell differentiation identified according to a method of the invention with a suitable diluent or carrier.

The use of a reporter gene construct, a cell, a cell aggregate, tissue, organ, implant or transplant, a non-human animal, a composition, an array or the apparatus defined herein in drug discovery or pharmacokinetic or pharmacological profiling is also a preferred embodiment of the present invention. Hence, the means and methods of the present invention described hereinbefore can be used in a variety of applications including, but not limited to, "loss of function" assays with ES cells containing homozygous mutations of specific genes, "gain of function" assays with ES cells overexpressing exogenous genes, developmental analysis of teratogenic/embryotoxic compounds in vitro, pharmacological assays and the establishment of model systems for pathological cell functions, and application of differentiation and growth factors for induction of selectively differentiated cells which can be used as a source for tissue grafts; see for review, e.g., Guan et al., Altex 16 (1999), 135-141.

As described in the examples, for the expression of the secreted human placental alkaline phosphatase protein (SEAP), a particular promoter has been used, i.e. the promoter region of the mouse alpha myosin heavy chain gene; see NCBI GenBank Accession No. U71441 (SEQ ID NO: 1). In accordance with the present invention, it could be shown that this promoter region is particularly suited for cell type-specific expression of a reporter gene, especially for the purposes of the present invention. In addition, further experiments performed in accordance with the present invention surprisingly revealed that the mouse ventricular myosin regulatory light chain gene promoter (see NCBI GenBank Accession No. AF302688; SEQ ID NO: 2) is particularly suited for the expression of the reporter gene as well. Thus, the present invention also relates to a recombinant DNA molecule as described hereinbefore in a cell type-specific reporter system, comprising the promoter of the mouse alpha myosin heavy chain gene and/or of the mouse ventricular myosin regulatory light chain gene, in particular the sequence depicted in NCBI GenBank Accession No. U71441 (SEQ ID NO: 1) and AF302688 (SEQ ID NO: 2), respectively, or a fragment thereof. Typically, the recombinant DNA molecule is a vector and comprises other functional DNA sequences such as ori, bacterial selectable marker, polyadenylation signal, multiple cloning sites, and the like. Hence, such vector may also be used to express specifically for example growth factors and the like or target cDNAs in order to elucidate their function during cell development. Thus, the present invention involves the use of the mentioned promoter regions for the expression of a DNA sequence encoding a reporter protein or a selectable marker; see supra. In a particular, the present invention relates to the expression vector described in Example 1 comprising the nucleotide sequence depicted in SEQ ID NO: 3 as well as to a corresponding vector, wherein the mouse alpha myosin heavy chain gene promoter has been replaced by the mouse ventricular myosin regulatory light chain gene promoter, and their use in the afore-described embodiments.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example, the public database "medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. A more complete understanding can be obtained by reference to the following specific examples and figure which are provided herein for purposes of illustration only and are not intended to limit the scope of the invention. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

The practice of the present invention will employ, unless otherwise indicated, conventional techniques of cell biology, cell culture, molecular biology, transgenic biology, microbiology, recombinant DNA, and immunology, which are within the skill of the art.

For further elaboration of general techniques concerning stem cell technology, the practitioner can refer to standard textbooks and reviews, for example Teratocarcinomas and embryonic stem cells: A practical approach (E. J. Robertson, ed., IRL Press Ltd. 1987); Guide to Techniques in Mouse Development (P. M. Wasserman et al., eds., Academic Press 1993); Embryonic Stem Cell Differentiation in Vitro (Wiles, Meth. Enzymol. 225 (1993), 900,); Properties and uses of Embryonic Stem Cells: Prospects for Application to Human Biology and Gene Therapy (Rathjen et al., Reprod. Fertil. Dev. 10 (1998), 31,). Differentiation of stem cells is reviewed in Robertson, Meth. Cell Biol. 75 (1997), 173; and Pedersen, Reprod. Fertil. Dev. 10 (1998), 31. Besides the sources for stem cells described already above further references are provided; see Evans and Kaufman, Nature 292 (1981), 154-156; Handyside et al., Roux's Arch. Dev. Biol., 196 (1987), 185-190; Flechon et al., J. Reprod. Fertil. Abstract Series 6 (1990), 25; Doetschman et al., Dev. Biol. 127 (1988), 224-227; Evans et al., Theriogenology 33 (1990), 125-128; Notarianni et al., J. Reprod. Fertil. Suppl., 43 (1991), 255-260; Giles et al., Biol. Reprod. 44 (Suppl. 1) (1991), 57; Strelchenko et al., Theriogenology 35 (1991), 274; Sukoyan et al., Mol. Reprod. Dev. 93 (1992), 418-431; Iannaccone et al., Dev. Biol. 163 (1994), 288-292.

Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (D. N. Glover ed., 1985); Oligonucleotide Synthesis (M. J. Gait ed., 1984); Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Gene Transfer Vectors for Mammalian Cells (Miller & Calos, eds.); Current Protocols in Molecular Biology and Short Protocols in Molecular Biology, 3rd Edition (F. M. Ausubel et al., eds.); and Recombinant DNA Methodology (R. Wu ed., Academic Press). Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.); Immobilized Cells And Enzymes (IRL Press, 1986); B. Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251). Other observations about the media and their impact on the culture environment have been made by Marshall McLuhan and Fred Allen.

### EXAMPLES

### Example 1: Generation of a vector for cardiac-specific SEAP expression

Using standard cloning procedures, a SEAP expression vector was obtained in the following way. A 5.4 kb fragment containing the promoter region of the α-mouse myosin heavy chain gene (Genbank No. U71441) was inserted into the polylinker of the pEGFP-1 vector (Clontech). The α-myosin heavy chain gene is specifically expressed by cardiac myocytes in differentiating ES cells (Gulick et al., J. Biol. Chem. 266 (1991), 9180-91855; Palermo et al., Cell Mol. Biol. Res. 41 (1995), 501-519; Morkin, Microsc. Res. Tech. 50 (2000), 522-531; Boheler et al., Circ. Res. 91 (2002), 189-201). Subsequently, the EGFP encoding sequence was removed and replaced by a SEAP encoding sequence (Berger et al., Gene 66 (1988), 1-10).

The resulting vector, designated α-MHC-SEAP, thus contains the SEAP gene under the transcriptional control of a heart-specific promoter. Further features of the vector include the presence of a Kozak consensus sequence to allow efficient translation of the SEAP mRNA, the presence of SV40 polyadenylation signals directing proper processing of the SEAP mRNA, the presence of a neomycin resistance cassette consisting of the SV40 early promoter, the neomycin/kanamycin resistance gene of Tn5 and polyadenylation signals from the Herpes simplex virus thymidine kinase gene to allow selection of stably transfected ES cells, and the presence of a bacterial promoter upstream of this cassette to confer kanamycin resistance in E. coli.

### Example 2: Generation of ES cell transfectants

The α-MHC-SEAP vector was linearized by restriction endonuclease digestion and introduced into D3 mouse embryonic stem (ES) cells (American Type Culture Collection) by electroporation. Following neomycin selection, single ES cell colonies were isolated and expanded. ES cell culture and transfection as well as selection of stable transfectants was done using standard procedures (Torres and Kühn, Laboratory protocols for conditional gene targeting. Oxford University Press, (1997) New York).

### Example 3: Differentiation of ES cells into cardiac cells in the presence of retinoic acid

The presence of excessive retinoic acid has striking effects on early heart development *in vivo* (Osmond et al., Development 113 (1991), 1405-1417; Yutzey et al., Development 120 (1994), 871-883; Dickman and Smith, Dev. Dyn. 206 (1996), 39-48; Drysdale et al., Dev. Biol. 188 (1997), 205-215). Furthermore, all-*trans* retinoic acid (CAS No. 302-79-4) was shown to inhibit the generation of cardiomyocytes from ES cells *in vitro* (Seiler et al. ALTEX 19 Suppl. 1 (2002), 55-63). Therefore, ES cells stably transfected with the α-MHC-SEAP vector were treated with retinoic acid at different concentrations and subjected to a differentiation protocol that resulted in the generation of cardiac cells. If secreted reporter, here SEAP, activity can indeed be used to quantify ES cell-derived cardiomyocytes, the amount of SEAP activity in culture supernatants should correlate inversely with the concentrations of retinoic acid present in the cultures.

All incubations of cell cultures were performed at 37 °C in a humidified atmosphere containing 5 % CO₂.

### ES cell aggregation

ES cells stably transfected with the α-MHC-SEAP vector (see Example 2) were trypsinized to obtain a single cell suspension. Cells were collected by centrifugation (800 g for 5 min) and resuspended in Iscove's Modified Dulbecco's Medium (IMDM, Invitrogen) supplemented with 20 % (v/v) fetal bovine serum (FBS, Invitrogen) at a density of 2x10⁶ cells/ml. To aggregate ES cells, 4 ml of this suspension were incubated on a 6 cm bacteriological dish (Greiner) on a rocking platform (GFL 3006) with agitation (50 rpm). After 6 hours, 2 ml of that culture were added to 13 ml IMDM containing 20 % FBS in a T25 flask (Falcon). The flask was incubated with rocking (50 rpm; GFL 3006) for 18 hours. During this procedure ES cell aggregates (embryoid bodies; EBs) were formed.

### ES cell differentiation in the presence of retinoic acid

All-*trans* retinoic acid (Sigma) was dissolved in dimethyl sulfoxide (DMSO, Sigma) to obtain 10⁻⁴ M, 10⁻⁵ M, 10⁻⁶ M, 10⁻¹⁷ M, 10⁻⁸ M, and 10⁻⁹ M stock solutions. In 10 cm petri dishes (bacteriological grade, Greiner) medium was prepared consisting of IMDM supplemented with 20 % FBS and retinoic acid added from above stocks to yield final concentrations of 10⁻⁷ M, 10⁻⁸ M, 10⁻⁹ M, 10⁻¹⁰ M, 10⁻¹¹ M, and 10⁻¹² M retinoic acid, respectively. One dish received DMSO (final concentration 0.1 % (v/v)) without retinoic acid (solvent control). Each dish contained 10 ml of medium. 70 EBs were transferred into each petri dish and incubated for 4 days.

Subsequently, EBs were transferred to 6-well plates (tissue culture grade; Becton-Dickinson). Each well contained 3 ml IMDM with 20 % FBS and retinoic acid and DMSO as described above for the culture in 10 cm dishes. 2 wells were set up for each concentration of retinoic acid and 2 wells as solvent controls. To each well 20 EBs were transferred from the respective 10 cm dish. Plates were incubated for 7 days. Subsequently, culture medium was exchanged with 2 ml knockout™ D-MEM (Invitrogen) supplemented with 20 % (v/v) knockout™ SR (Invitrogen). Samples of cell culture supernatants were collected after incubation of EBs for further two days.

### Determination of SEAP activity

Samples were assayed for SEAP activity using the Great EscAPe SEAP Fluorescence Detection Kit (Becton-Dickinson) following the manufacturer's instructions. Fluorescence was measured using a microplate reader (Safire, Tecan). Background activity was determined by assaying a sample of fresh medium (knockout™ D-MEM supplemented with 20 % (v/v) knockout™ SR).

Results are shown in Fig. 1. Concentrations of retinoic acid below 10⁻⁹ M had no influence on SEAP activity as compared to controls. SEAP activity decreased sharply at 10⁻⁸ M retinoic acid. At higher concentrations no activity was observed.

Thus, a concentration-dependent effect of retinoic acid on SEAP activity in culture supernatants is clearly observed. Moreover, these data are consistent with the concentration-dependent inhibition of cardiomyocyte generation by retinoic acid described previously (Seiler et al., 2002, supra). Therefore, the results indicate the suitability of the reporter system presented here to quantify ES cell-derived cardiomyocytes and its usefulness for the monitoring of the differentiation process of ES cells.

## Claims

1. A method of monitoring cell differentiation comprising:
(a) culturing cells capable of differentiating into at least one particular cell type containing at least one recombinant nucleic acid molecule comprising a reporter gene encoding a product that is secreted upon cell differentiation, or maintaining a non-human animal comprising such cells, under conditions allowing differentiation of the cells; and
(b) quantifying the differentiated cells by determining the amount or activity of the reporter gene product either within a body fluid of said transgenic non-human animal or the cell culture medium.

2. The method of claim 1, wherein said recombinant nucleic acid molecule comprises at least one cell type-specific regulatory sequence operably linked to said reporter gene.

3. The method of claim 1 or 2, wherein said cells are or are derived from stem cells.

4. The method of claim 3, wherein said stem cells are cells of an embryonic stem cell line or multipotent adult progenitor cells (MAPCs).

5. The method of any one of claims 1 to 4, wherein said reporter gene product comprises a secretory leader sequence.

6. The method of any one of claims 2 to 5, wherein said regulatory sequence comprises a promoter and/or enhancer element.

7. The method of any one of claims 1 to 6, wherein said cell type is selected from the group consisting of connecting fibroblasts, stromal cells, endothelial cells, glial cells, neural cells, neuronal cells, hematopoietic cells, smooth muscle cells, skeletal muscle cells, epithelial cells, and cardiac cells.

8. The method of claim 6 or 7, wherein said promoter or enhancer is selected from the group consisting of aMHC, MLC2V, VE-cadherin, Tie-2, Flk-1, Flt-1, GFAP, alpha-1-tubulin and collagen 2 promoter or enhancer.

9. The method of any of claims 1 to 8, wherein said reporter gene product is secreted alkaline phosphatase (SEAP) or alpha-amylase.

10. The method of any one of claims 1 to 9, wherein said recombinant nucleic acid molecule further comprises a selectable marker expressed by multi- or pluripotent cells.

11. The method of any one of claims 1 to 10, wherein said cells form cell aggregates or tissue-like aggregates derived from different cell types.

12. The method of any one of claims 1 to 11, wherein said cells form embryoid bodies (EBs).

13. A reporter gene construct for monitoring cell differentiation by quantifying the differentiated cells comprising a recombinant nucleic acid molecule as defined in any one of claims 1, 2, 5, 6 or 8 to 10.

14. A cell comprising a reporter gene construct of claim 13, wherein said cell is capable of differentiating into at least one particular cell type.

15. A cell aggregate of at least one cell type comprising cells of claim 14.

16. A tissue comprising cells of claim 14 or a cell aggregate of claim 15.

17. An organ comprising a tissue of claim 16, a cell of claim 14 or a cell aggregate of claim 15.

18. An implant or transplant comprising an organ of claim 17, a tissue of claim 16, a cell of claim 14 or a cell aggregrate of claim 15.

19. A non-human animal comprising a reporter gene construct of claim 13, a cell of claim 14, a cell aggregate of claim 15, a tissue of claim 16 or an organ of claim 17.

20. A composition of matter comprising a reporter gene construct of claim 13, a tissue of claim 16, cells of claim 14 or a cell aggregate of claim 15.

21. An array comprising a solid support and attached thereto or suspended thereon cells of claim 14, a cell aggregate of claim 15 or a tissue of claim 16.

22. Use of an apparatus for analyzing the array of claim 21.

23. A method of obtaining and/or profiling a modulator of cell differentiation comprising:
(a) contacting a test sample comprising a cell of claim 14, a cell aggregate of claim 15, a tissue of claim 16 or an organ of claim 17 or a non-human animal of claim 19 with a test substance; and
(b) determining the effect of the test substance on the amount of the reporter gene product or activity compared to a control sample or animal.

24. The method of any one of claims 1 to 12 or 23, wherein a compound known to activate or inhibit the differentiation process is added to the culture medium or animal.

25. The method of any one of claims 23 or 24, wherein the test substance is a therapeutic agent, or a mixture of therapeutic agents.

26. The method of any one of claims 23 to 25, which is performed on an array.

27. The method of any one of claims 1 to 12 or 23 to 26, wherein said one or more cells are genetically engineered to (over)express or inhibit the expression of a target gene.

28. A kit useful for conducting a method of any one of claims 1 to 12 or 23 to 31, containing a reporter gene construct of claim 13 or a cell of claim 14, and optionally standard compounds, like cell culture media, selection agents, detection agents for the reporter molecule and control samples.

29. Use of a reporter gene construct of claim 13, a cell of claim 14, a cell aggregate of claim 15, a tissue of claim 16, an organ of claim 17, the implant or transplant of claim 18, a non-human animal of claim 19, the composition of claim 20 or an array of claim 21 in drug discovery or pharmacokinetic or pharmacological profiling.

30. A vector comprising the promoter region of the mouse alpha myosin heavy chain gene or of the ventricular myosin regulatory light chain gene, and operably linked thereto a 5 heterologous DNA sequence, which encodes secreted alkaline phosphatase protein (SEAP).

31. The vector of claim 35, wherein said promoter comprises the nucleotide sequence of SEQ ID NO: 1 or 2, or a fragment thereof.

32. The vector of claim 35 or 36 comprising the nucleotide sequence of SEQ ID NO: 3.

33. Use of a promoter region of the mouse alpha myosin heavy chain gene or of the ventricular myosin regulatory light chain gene for the specific expression of heterologous DNA sequences during embryogenesis or cell development in a method of any one of claims 1 to 12 or 23 to 31.

## Patentansprüche

1. Verfahren zur Überwachung der Zelldifferenzierung umfassend:
(a) das Kultivieren von Zellen, die dazu in der Lage sind, zu mindestens einem bestimmten Zelltyp zu differenzieren, der mindestens ein rekombinantes Nukleinsäuremolekül enthält, das ein Reportergen umfasst, das für ein Produkt kodiert, das bei der Zelldifferenzierung sezerniert wird, oder die Aufrechterhaltung eines nicht humanen Tiers, das solche Zellen umfasst, unter Bedingungen, die eine Differenzierung der Zellen gestatten; und
(b) das Quantifizieren der differenzierten Zellen durch Bestimmen der Menge oder der Aktivität des Reportergenprodukts entweder in einer Körperflüssigkeit des transgenen nicht humanen Tiers oder im Zellkulturmedium.

2. Verfahren nach Anspruch 1, wobei das rekombinante Nukleinsäuremolekül mindestens eine für den Zelltyp spezifische regulatorische Sequenz umfasst, die funktionell mit dem Reportergen verknüpft ist.

3. Verfahren nach Anspruch 1 oder 2,wobei die Zellen Stammzellen oder von Stammzellen abgeleitete Zellen sind.

4. Verfahren nach Anspruch 3, wobei die Stammzellen Zellen aus einer embryonalen Stammzelllinie oder multipotente adulte Progenitorzellen (MAPC) sind.

5. Verfahren nach einem der Ansprüche 1 bis 4, wobei das Reportergenprodukt eine sekretorische Leadersequenz umfasst.

6. Verfahren nach einem der Ansprüche 2 bis 5, wobei die regulatorische Sequenz ein Promotor- und/oder ein Enhancerelement umfasst.

7. Verfahren nach einem der Ansprüche 1 bis 6, wobei der Zelltyp ausgewählt ist aus der Gruppe bestehend aus Bindegewebsfibroblasten, Stromazellen, Endothelzellen, Gliazellen, neuralen Zellen, neuronalen Zellen, hämatopoetischen Zellen, glatten Muskelzellen, Skelettmuskelzellen, Epithelzellen und kardialen Zellen.

8. Verfahren nach Anspruch 6 oder 7, wobei der Promotor oder Enhancer ausgewählt ist aus der Gruppe bestehend aus einem aMHC-, MLC2V-, VE-Cadherin-, Tie-2-, Flk-1-, Flt-1-, GFAP-, alpha-1-Tubulin- und Collagen 2-Promotor oder Enhancer.

9. Verfahren nach einem der Ansprüche 1 bis 8, wobei das Reportergenprodukt sezernierte alkalische Phosphatase (SEAP) oder alpha-Amylase ist.

10. Verfahren nach einem der Ansprüche 1 bis 9, wobei das rekombinante Nukleinsäuremolekül des Weiteren einen selektierbaren Marker umfasst, der durch multi- oder pluripotente Zellen exprimiert wird.

11. Verfahren nach einem der Ansprüche 1 bis 10, wobei die Zellen Zellaggregate oder gewebeähnliche Aggregate bilden, die von unterschiedlichen Zelltypen abgeleitet sind.

12. Verfahren nach einem der Ansprüche 1 bis 11, wobei die Zellen Embryoidkörper (EB) bilden.

13. Reportergenkonstrukt zur Überwachung der Zelldifferenzierung durch Quantifizieren der differenzierten Zellen, umfassend ein rekombinantes Nukleinsäuremolekül wie in einem der Ansprüche 1, 2, 5, 6 oder 8 bis 10 definiert.

14. Zelle umfassend ein Reportergenkonstrukt nach Anspruch 13, wobei die Zelle dazu in der Lage ist, zu mindestens einem bestimmten Zelltyp zu differenzieren.

15. Zellaggregat mindestens eines Zelltyps umfassend Zellen nach Anspruch 14.

16. Gewebe umfassend Zellen nach Anspruch 14 oder ein Zellaggregat nach Anspruch 15.

17. Organ umfassend ein Gewebe nach Anspruch 16, eine Zelle nach Anspruch 14 oder ein Zellaggregat nach Anspruch 15.

18. Implantat oder Transplantat umfassend ein Organ nach Anspruch 17, ein Gewebe nach Anspruch 16, eine Zelle nach Anspruch 14 oder ein Zellaggregat nach Anspruch 15.

19. Nicht humanes Tier umfassend ein Reportergenkonstrukt nach Anspruch 13, eine Zelle nach Anspruch 14, ein Zellaggregat nach Anspruch 15, ein Gewebe nach Anspruch 16 oder ein Organ nach Anspruch 17.

20. Stoffzusammensetzung umfassend ein Reportergenkonstrukt nach Anspruch 13, ein Gewebe nach Anspruch 16, Zellen nach Anspruch 14 oder ein Zellaggregat nach Anspruch 15.

21. Array umfassend einen festen Träger und entweder daran befestigt oder darauf suspendiert Zellen nach Anspruch 14, ein Zellaggregat nach Anspruch 15 oder ein Gewebe nach Anspruch 16.

22. Verwendung einer Vorrichtung zur Analyse des Arrays nach Anspruch 21.

23. Verfahren zum Erhalt und/oder zur Profilierung eines Modulators der Zelldifferenzierung, umfassend:
(a) Inkontaktbringen einer Testprobe umfassend eine Zelle nach Anspruch 14,ein Zellaggregat nach Anspruch 15, ein Gewebe nach Anspruch 16 oder ein Organ nach Anspruch 17 oder ein nicht humanes Tier nach Anspruch 19 mit einer Testsubstanz; und
(b) Bestimmen der Wirkung der Testsubstanz auf die Menge an Reportergenprodukt oder die Aktivität im Vergleich zu einer Kontrollprobe oder einem Kontrolltier.

24. Verfahren nach einem der Ansprüche 1 bis 12 oder 23, wobei eine Verbindung, von der bekannt ist, dass sie den Differenzierungsprozess aktiviert oder inhibiert, zu dem Kulturmedium oder dem Tier gegeben wird.

25. Verfahren nach einem der Ansprüche 23 oder 24, wobei die Testsubstanz ein therapeutischer Wirkstoff oder ein Gemisch von therapeutischen Wirkstoffen ist.

26. Verfahren nach einem der Ansprüche 23 bis 25, das auf einem Array durchgeführt wird.

27. Verfahren nach einem der Ansprüche 1 bis 12 oder 23 bis 26, wobei die eine oder die mehreren Zellen gentechnisch verändert wird bzw. werden, um ein Zielgen zu (über-) exprimieren oder die Expression eines Zielgens zu inhibieren.

28. Kit, das zweckmäßig für die Durchführung eines Verfahrens nach einem der Ansprüche 1 bis 12 oder 23 bis 31 ist, enthaltend ein Reportergenkonstrukt nach Anspruch 13 oder eine Zelle nach Anspruch 14 sowie gegebenenfalls Standardverbindungen wie Zellkulturmedien, Agenzien zur Selektionierung, Agenzien zur Detektion des Reportermoleküls und Kontrollproben.

29. Verwendung eines Reportergenkonstrukts nach Anspruch 13, einer Zelle nach Anspruch 14, eines Zellaggregats nach Anspruch 15, eines Gewebes nach Anspruch 16, eines Organs nach Anspruch 17, des Implantats oder Transplantats nach Anspruch 18, eines nicht humanen Tiers nach Anspruch 19, der Zusammensetzung nach Anspruch 20 oder eines Arrays nach Anspruch 21 in der Arzneimittelforschung oder der pharmakokinetischen oder pharmakologischen Profilierung.

30. Vektor umfassend die Promotorregion des murinen Gens für die schwere Kette von alpha-Myosin oder des Gens für die regulatorische leichte Kette von ventrikulärem Myosin, sowie funktionell mit dieser verknüpft eine heterologe DNA-Sequenz, die für das Protein der sezernierten alkalischen Phosphatase (SEAP) kodiert.

31. Vektor nach Anspruch 30, wobei der Promotor die Nukleotidsequenz gemäß SEQ ID NR: 1 oder 2 oder ein Fragment davon umfasst.

32. Vektor nach Anspruch 30 oder 31 umfassend die Nukleotidsequenz gemäß SEQ ID NR: 3.

33. Verwendung einer Promotorregion des murinen Gens für die schwere Kette von alpha-Myosin oder des Gens für die regulatorische leichte Kette von ventrikulärem Myosin für die spezifische Expression von heterologen DNA-Sequenzen während der Embryogenese oder der Zellentwicklung in einem Verfahren nach einem der Ansprüche 1 bis 12 oder 23 bis 27.

## Revendications

1. Procédé pour contrôler la différenciation de cellules comprenant :
(a) la mise en culture de cellules aptes à se différencier en au moins un type cellulaire particulier contenant au moins une molécule d'acide nucléique recombinante comprenant un gène rapporteur codant un produit qui est sécrété lors de la différenciation des cellules, ou l'entretien d'un animal non humain comprenant de telles cellules, dans des conditions permettant la différenciation des cellules ; et
(b) la quantification des cellules différenciées par détermination de la quantité ou de l'activité du produit du gène rapporteur soit dans un fluide corporel dudit animal non humain transgénique soit dans le milieu de culture des cellules.

2. Procédé selon la revendication 1, dans lequel ladite molécule d'acide nucléique recombinante comprend au moins une séquence régulatrice spécifique d'un type cellulaire fonctionnellement liée audit gène rapporteur.

3. Procédé selon la revendication 1 ou 2, dans lequel lesdites cellules sont des cellules souches ou dérivent de cellules souches.

4. Procédé selon la revendication 3, dans lequel lesdites cellules souches sont des cellules d'une lignée de cellules souches embryonnaires ou des cellules progénitrices adultes multipotentes (MAPC).

5. Procédé selon l'une quelconque des revendications 1 à 4, dans lequel ledit produit du gène rapporteur comprend une séquence de tête sécrétoire.

6. Procédé selon l'une quelconque des revendications 2 à 5, dans lequel ladite séquence régulatrice comprend un élément promoteur et/ou activateur.

7. Procédé selon l'une quelconque des revendications 1 à 6, dans lequel ledit type cellulaire est choisi dans le groupe constitué des fibroblastes du tissu conjonctif, des cellules stromales, des cellules endothéliales, des cellules gliales, des cellules neurales, des cellules neuronales, des cellules hématopoïétiques, des cellules du muscle lisse, des cellules du muscle squelettique, des cellules épithéliales, et des cellules cardiaques.

8. Procédé selon la revendication 6 ou 7, dans lequel ledit promoteur ou activateur est choisi dans le groupe constitué du promoteur ou de l'activateur de aMHC, de MLC2V, de VE-cadhérine, de Tie-2, de Flk-1, de Flt-1, de GFAP, de l'alpha-1-tubuline et du collagène de type 2.

9. Procédé selon l'une quelconque des revendications 1 à 8, dans lequel ledit produit du gène rapporteur est la phosphatase alcaline sécrétée (SEAP) ou l'alpha-amylase.

10. Procédé selon l'une quelconque des revendications 1 à 9, dans lequel ladite molécule d'acide nucléique recombinante comprend en outre un marqueur de sélection exprimé par des cellules multi- ou pluripotentes.

11. Procédé selon l'une quelconque des revendications 1 à 10, dans lequel lesdites cellules forment des agrégats cellulaires ou des agrégats d'aspect tissulaire issus de différents types cellulaires.

12. Procédé selon l'une quelconque des revendications 1 à 11, dans lequel lesdites cellules forment des corps embryoïdes (EB).

13. Construction de gène rapporteur servant à contrôler la différenciation de cellules par quantification des cellules différenciées comprenant une molécule d'acide nucléique recombinante telle que définie dans l'une quelconque des revendications 1, 2, 5, 6 ou 8 à 10.

14. Cellule comprenant une construction de gène rapporteur selon la revendication 13, où ladite cellule est apte à se différencier en au moins un type cellulaire particulier.

15. Agrégat cellulaire d'au moins un type cellulaire comprenant des cellules selon la revendication 14.

16. Tissu comprenant des cellules selon la revendication 14 ou un agrégat cellulaire selon la revendication 15.

17. Organe comprenant un tissu selon la revendication 16, une cellule selon la revendication 14, ou un agrégat cellulaire selon la revendication 15.

18. Implant ou transplant comprenant un organe selon la revendication 17, un tissu selon la revendication 16, une cellule selon la revendication 14 ou un agrégat cellulaire selon la revendication 15.

19. Animal non humain comprenant une construction de gène rapporteur selon la revendication 13, une cellule selon la revendication 14, un agrégat cellulaire selon la revendication 15, un tissu selon la revendication 16 ou un organe selon la revendication 17.

20. Composition de matière comprenant une construction de gène rapporteur selon la revendication 13, un tissu selon la revendication 16, des cellules selon la revendication 14 ou un agrégat cellulaire selon la revendication 15.

21. Puce comprenant un support solide et, fixées à celui-ci ou en suspension sur celui-ci, des cellules selon la revendication 14, un agrégat cellulaire selon la revendication 15, ou un tissu selon la revendication 16.

22. Utilisation d'un appareil pour analyser la puce selon la revendication 21.

23. Procédé d'obtention et/ou de profilage d'un modulateur de la différenciation cellulaire, comprenant :
(a) la mise en contact d'un échantillon d'essai comprenant une cellule selon la revendication 14, un agrégat cellulaire selon la revendication 15, un tissu selon la revendication 16, un organe selon la revendication 17 ou un animal non humain selon la revendication 19, avec une substance à tester ; et
(b) la détermination de l'effet de la substance à tester sur la quantité du produit du gène rapporteur ou le niveau d'activité de celui-ci par rapport à un échantillon ou animal témoin.

24. Procédé selon l'une quelconque des revendications 1 à 12 ou 23, dans lequel un composé connu pour activer ou inhiber le processus de différenciation est ajouté au milieu de culture ou à l'animal.

25. Procédé selon l'une quelconque des revendications 23 ou 24, dans lequel la substance à tester est un agent thérapeutique, ou un mélange d'agents thérapeutiques.

26. Procédé selon l'une quelconque des revendications 23 à 25, qui est mis en oeuvre sur une puce.

27. Procédé selon l'une quelconque des revendications 1 à 12 ou 23 à 26, dans lequel ladite/lesdites une ou plusieurs cellule(s) est/sont génétiquement manipulée(s) pour (sur)exprimer ou inhiber l'expression d'un gène cible.

28. Kit utile pour mettre en oeuvre un procédé selon l'une quelconque des revendications 1 à 12 ou 23 à 27, contenant une construction de gène rapporteur selon la revendication 13 ou une cellule selon la revendication 14, et, facultativement, des composés standard tels que des milieux de culture cellulaire, des agents de sélection, des agents de détection pour la molécule rapporteur et des échantillons témoins.

29. Utilisation d'une construction de gène rapporteur selon la revendication 13, d'une cellule selon la revendication 14, d'un agrégat cellulaire selon la revendication 15, d'un tissu selon la revendication 16, d'un organe selon la revendication 17, de l'implant ou du transplant selon la revendication 18, d'un animal non humain selon la revendication 19, de la composition selon la revendication 20 ou d'une puce selon la revendication 21, dans le cadre de la découverte de médicaments ou de l'établissement de profils pharmacocinétiques ou pharmacologiques.

30. Vecteur comprenant la région promotrice du gène de la chaîne lourde alpha de la myosine murine ou du gène de la chaîne légère régulatrice de la myosine ventriculaire et, fonctionnellement liée à ladite région promotrice, une séquence d'ADN hétérologue, qui code la protéine phosphatase alcaline sécrétée (SEAP).

31. Vecteur selon la revendication 30, dans lequel ledit promoteur comprend la séquence nucléotidique de SEQ ID NO: 1 ou 2, ou un fragment de celle-ci.

32. Vecteur selon la revendication 30 ou 31, comprenant la séquence nucléotidique de SEQ ID NO: 3.

33. Utilisation d'une région promotrice du gène de la chaîne lourde alpha de la myosine murine ou du gène de la chaîne légère régulatrice de la myosine ventriculaire pour l'expression spécifique de séquences d'ADN hétérologues au cours de l'embryogenèse ou du développement cellulaire, dans un procédé selon l'une quelconque des revendications 1 à 12 ou 23 à 27.
